# EUROPEAN PATENT SPECIFICATION

(11) **EP 1 731 175 B1**
(45) Date of publication and mention of the grant of the patent: **30.09.2009**
(21) Application number: 06013191.9
(22) Date of filing: 12.11.1999
(51) Int. Cl.: A61L 15/28, A61L 26/00

(54) **Hemostatic cross-linked dextran beads useful for rapid blood coagulation and hemostatis**
Hämostatische vernetzte Dextranperlen verwendbar zur schnellen Blutgerinnung und Hämostase
Perles de dextrane réticulés hemostatiques permettant une coagulation sanguine rapide et l'hémostase

(30) Priority: 12.11.1998 US 108185 P; 13.04.1999 US 290846
(43) Date of publication of application: 13.12.2006
(62) Divisional of application: 99958903.9
(73) Proprietor: International Manufacturing Group, Inc., West Sacramento CA 95605 (US); Theis, Jerold H., Davis, CA 95616 (US)
(72) Inventor: Cochrum, Kent, C., Davis CA 95616 (US); Gunther, Robert, A., Davis CA 95616 (US); Jemtrud, Susan, A., San Francisco CA 95616 (US); Beninsig, Franklin, M., Sacramento CA 95814 (US)
(74) Representative: Wichmann, Hendrik

(56) References cited:
- EP-A1- 0 613 693
- GB-A- 942 305
- GB-A- 1 454 055
- GB-A- 1 460 607
- US-A- 974 054
- US-A- 983 073
- US-A- 2 914 444
- US-A- 4 793 336

## Description

### BACKGROUND OF THE INVENTION

The present invention relates to a novel hemostatic polymer composition as defined in the claims. Further, a composition is described comprising of a substance containing uncharged organic hydroxyl groups and a substance containing at least one of a halogen atom and/or an epoxy group. The polymer is especially useful for the rapid induction of blood coagulation and hemostasis at a wound or bleeding site. Methods of using the hemostatic polymer are also provided.

### Field of the Invention:

Wound healing refers to a complex series of biochemical and cellular events, which result in the contracting, closing and healing of a wound, which, in itself, is a traumatic insult to the integrity of a tissue. Wound management, contemplates protecting the wound from additional trauma and/or environmental factors that may delay the healing process. Towards this end, it advocates a combined systemic and local approach to facilitate wound healing, which includes the use of antibiotics and the application of a suitable dressing.

The principal function of a wound dressing is to provide an optimum healing environment by mimicking a natural barrier function of the epithelium. Accordingly, in practice, a wound dressing should, at a minimum:
i) control bleeding,
ii) isolate and protect the wound or bleeding site from the external environment before healing can begin
iii) prevent further contamination or infection and
iv) maintain a moist micro-environment next to the wound surface.

It is well accepted that wound healing may be impeded by an infection, at the wound or bleeding site, because it facilitates further tissue damage and promotes inflammation. Such contamination may result from contact with an infected object or the ingress of dirt, dust, or microorganism, either at the time of injury or later from the subject's own skin. As consequence, subsequent further wound repair is hampered by the progression of inflammation consisting of vascular leakage, the release and activation of lytic enzymes, free radical generation, oxygen consumption, and the sensitization of tissue nerve endings. Thus measures to limit inflammation should promote wound healing provided that such measures do not compromise the tissue's ability to resist infection and essential macrophage function.

The control of topical bleeding is also of critical importance in wound management, especially in the armed forces as well as in civilian use such as trauma treatment and the general administration of first aid. While attempts at controlling bleeding have been proposed, as explained below, conventional methods for controlling bleeding are fraught with numerous drawbacks.

A conventional method of controlling topical bleeding including external hemorrhage advocates the use of cotton gauze pads capable of absorbing 250 ml of blood. Such use is very common in the armed forces and particularly in civilian trauma units. However, cotton pads are generally considered passive dressings, because of their inability to initiate or accelerate blood clotting.

Another method of controlling bleeding (i.e., wound closure) advocates the use of surgical sutures and staples. Sutures are recognized to provide adequate wound support; however, sutures cause additional trauma to the wound site (by reason of the need for the needle and suture to pass through tissue) and are time-consuming to place, and, at skin level, can cause unattractive wound closure marks. Surgical staples have been developed to speed wound apposition and provide improved cosmetic results, these are known to impose additional wound trauma and require the use of ancillary and often expensive devices for positioning and applying them.

Wound healing is a complex process involving such factors as cells, extracellular matrix (ECM) components and the cellular microenvironment. Essentially, all wound healing involves the repair or replacement of damaged tissues. The precise nature of such repair or replacement depends upon the tissues involved, although all such processes involve certain basic principles.

By way of background, as a part of hemostasis, clot formation is often a life-saving process in response to trauma and serves to arrest the flow of blood from severed vasculature. In addition, it is often desirable to initiate or enhance the body's natural hemostatic process. For example, after severe trauma, a victim may require supplemental assistance in stopping bleeding or hemorrhage caused by the trauma.

Blood coagulation occurs by means of a complex cascade of reactions called the coagulation cascade which involves the formation of the enzyme thrombin, which is formed from prothrombin via the interactions of factor Xa, calcium and other ancillary substances. For an excellent review of the blood coagulation cascade, the reader is directed to the article by Mann, K.G., XVII Congress of the International Society on Thrombosis and Haemostasis, Medscape, 1999.

In wound healing, the final stage of the coagulation cascade results in the formation of insoluble fibrin, which forms the insoluble structure of the blood clot. The fibrin is formed from fibrinogen in the presence of other plasma components, most notably, thrombin and factor XIII ,
wherein the thrombin converts fibrinogen and factor XIII into their reactive forms.

Thrombin does not exist in an active state within the blood circulation system but rather in the form of an inactive precursor, prothrombin. Thrombin is activated, however, through one of two mechanisms commonly referred to as the extrinsic and intrinsic pathways. The intrinsic pathway activates thrombin when blood contacts glass outside the body, as in a test tube or other negatively charged surfaces. The extrinsic pathway, on the other hand, activates thrombin when blood comes in contact with injured tissues, which produce tissue thromboplastin.

Over the course of the past decade, a better understanding of the wound healing process together with improvements in modern surgical suturing techniques have greatly improved wound treatment. Such improvement have, in turn, advanced the use of suitable supplementary materials, such as fibrin glues, sealants or adhesives, to accelerate hemostasis as well as to optimize conditions and control of wound closure. Also included are proposals for using thrombin in the management of a wound.

The use of exogenous thrombin as a clot-enhancing or hemostatic agent is known in the art. For example, thrombin has generally been used in surgery or in emergency situations. It is applied topically at the wound or bleeding site, generally in powder or solution form. However, the use of thrombin as a single agent for inducing clotting and hemostasis is limited to minor clots or injuries. It alone is often insuffcient and needs supplementation to be effective.

In more extensive bleeding or in hemorrhage, it is generally used on a matrix that holds the thrombin at the desired location thereby providing a structure for clot formation. Matrix materials known in the art include fibrin foam-like compositions and gelatinous sponges. However, even in conjunction with such matrix materials, thrombin is generally regarded as ineffective for inducing coagulation and hemostasis on arterial bleeding.

An alternative approach to the use of thrombin as an adjunct in inducing coagulation involves the application of thrombin along with fractionated plasma at the wound site.

Therapeutic compositions containing fibrinogen and thrombin for use as tissue or hemostatic agents, adhesives or sealants are known. See, Cronkite E. P. et al., J.A.M.A., 124, 976 (1944), Tidrick R. T. and Warner E. D., Surgery, 15, 90 (1944).

Plasma, as the name implies, refers to the liquid portion of the blood. The chief components of plasma are proteins, anions, and cations. The proteins include albumin and globulins. Anions are chiefly chloride and bicarbonate, while cations are largely sodium, potassium, calcium and magnesium. Blood plasma also circulates immunoglobulins and several of the essential components for clot formation.

Fractionated plasma is normally obtained from either autologous or nonautologous blood sources, several hours in advance of its need and is frozen, cryoprecipitated and then thawed before being combined with thrombin at the bleeding site.

An advantage associated with plasma from an autologous blood source is that its use obviates the concern for transmission of human viruses. However, a drawback associated with the use of such preparations includes unpredictable adhesive strength. In addition, the product may be available only in limited quantities and not be available on demand. As such, the use of fractionated plasma as a thrombin adjunct for promoting blood clotting is significantly hampered because the plasma must be obtained several hours and usually a day prior to its use. The problems are magnified when emergency situations arise and the several hour time lag for plasma fractionation is unavailable or otherwise impracticable.

Preferred donors for nonanalogous plasma are mammals other than humans. However, recent concerns with the use of blood products obtained from sources foreign to the patient have severely impeded the use of nonautologous plasma because of the risk of transmitting infectious diseases to the patient.

In view of the above, the prior art has proposed fibrinogen based therapies, which, like the thrombin based therapies, is also attended with numerous disadvantages.

Fibrinogen is a soluble protein found in the blood plasma of all vertebrates that when contacted by thrombin becomes polymerized to an insoluble gel-like network. In polymerized form, the fibrinogen is referred to as fibrin. The conversion of fibrinogen to fibrin is crucial to normal hemostasis in vertebrates.

Fibrinogen represents about 2 to 4 grams/liter of the blood plasma protein. The fibrinogen molecule is a monomer and has a molecular weight of about 340,000 and is a rod or ellipsoid-shaped particle. It has been determined that fibrinogen, in circulating form, consists of a dimec of 2 identical units each consisting of 3 polypeptides known as α, β and γ. "A" and "B" represent the two small aminotenninal peptides, known as fibrinopeptide A and fibrinopeptide B, respectively. The cleavage of fibrinopeptides A from fibrinogen in the transformation of fibrinogen by thrombin results in the fibrin I compound and the subsequent cleavage of fibrinopeptides B results in the fibrin II compound. Such cleavage of fibrinopeptides A and B reduces the molecular weight of fibrinogen by an extremely small amount, about 6,000 out of 340,000 daltons, but exposes the polymerization sites.

The fibrinogen protein contains numerous binding sites important to the final assembly of the fibrin network. For a detailed review of fibrinogen structure see Blomback, B., "Fibrinogen and Fibrin Formation and its Role in Fibrinolysis", Chapter 11, pp. 225-269, in Goldstein, J. ed., Biotechnology of Blood, Butterworth-Heinemann, Boston, Mass. 1991. For a review of the mechanisms of blood coagulation and the structure of fibrinogen, see C. M. Jackson, Ann. Rev. Biochem., 49:765-81 (1980) and B. Furie and B. C. Furie, Cell, 53:505-518 (1988).

Over the past decade, topical application of fibrin for the purposes of initiating hemostasis as a surface coagulant has resulted in the medical community referring to such use of fibrin as that of a "fibrin glue".

Fibrin glue is composed of a mixture of human fibrinogen and bovine thrombin. It is sold as a kit containing separate vials of fibrinogen and thrombin solutions. These solutions are mixed together and applied to the wound in various ways, including as a paste, as a spray or on a patch. Fibrin glue, however, is an inconsistent and ineffective therapy for hemostasis. The mixing, soaking, and coating of a patch with fibrin glue requires time-consuming and cumbersome procedures. Each of the preparation steps introduces potential errors and thus their efficacy varies with the experience of operating room personnel. Moreover, during the preparation of such solution, further hemorrhage occurs and the solutions are washed away by intense bleeding. Despite the headway made in fibrinogen compositions and surgical techniques, these pitfalls in achieving hemostasis underscore the need for development of a suitable product.

Also, the physical or chemical properties (for example, solubility) of this protein limit substantially its use. See U.S. Pat. No. 4,650,678, EP 085 923 B1, EP 085 923 B2, and EP 085 923 A1, all of which detail the difficulty in reconstituting fibrinogen from lyophilized material (the form of fibrinogen preferred for long term storage for clinical use). More, the '678 patent also notes that for a fibrinogen solution to be effective as an adhesive composition, the solution must contain about 80 mg/ml or more of clottable fibrinogen.

Fibrin glue (sealant, adhesive) is based on the basic physiological function of fibrinogen and has proven particularly advantageous over non-biological adhesives because fibrin-based glues mimic the natural coagulation cascade and enhance the healing process by imitating the final stages of coagulation, thereby facilitating the formation of a fibrin clot.

Conventional fibrin glue/sealants generally consist of concentrated human fibrinogen, bovine aprotinin and factor XIII, as the first component and bovine thrombin and calcium chloride as the second component. In the presence of calcium ions, activation of fibrinogen and fibrin-stabilizing factor XIII with thrombin produces a stable fibrin clot. The most common method of preparing fibrin glue is by simultaneously mixing concentrated fibrinogen complex obtained from pooled human blood, bovine thrombin and ionic calcium immediately before use. Alternatively, the components may be premixed to facilitate polymerization.

In general, when the components are applied to the tissue in sequence, fibrinogen solution is first applied onto the tissue. Thereafter, small amounts of a highly concentrated thrombin and/or factor XIII solution are applied to the tissue-supported fibrinogen solution to promote coagulation. Usually, a fibrinolysis inhibitor is also added in order to prevent premature lysis and premature dehiscence of the adapted tissue parts. However, this technique is very expensive and complicated because of the necessary separate preparation, storage and application of the individual components making up the adhesive. Additionally, the technique is time-consuming and difficult to control.

The addition of the nonhuman, typically bovine thrombin in the fibrin glue preparations used for treatments in humans has resulted in severe and even fatal anaphylactic reactions. Hemostasis abnormalities caused by antibodies to bovine proteins, such as bovine thrombin, which cross-react with human proteins, including thrombin and factor V have been reported in J. Thorac. Cardiovac. Surg., 105:892 (1993). Similarly, foreign body reactions following the use of these fibrin bovine thrombin containing glues have been detected and described in Eur. J. Pediatr. Surg., 2:285 (1992). It is well known that bovine thrombin is a carrier of the infectious agent bovine spongiform encephalitis (BSE) and other viruses pathogenic to mammals. Furthermore, bovine thrombin is a potent antigen, which can cause immunological reactions in humans. Thus, the use of bovine thrombin could result in the recipient of the bovine thrombin being adversely affected. See D. M. Taylor, J. of Hospital Infection, 18 (Supplement A) :141-146 (1991), S. B. Prusiner et al., Cornell Vet, 81 No. 2: 85-96 (1991) and D. Matthews, J. Roy. Soc. Health, 3-5 (February 1991).

In addition, the fibrinogen for use in the above fibrin glue is often concentrated from human plasma by cryoprecipitation and precipitation using various reagents, e.g., polyethylene glycol, ether, ethanol, ammonium sulfate or glycine. There always exists the risk of an immunogenic reaction to the fibrinogen component of traditional fibrin glue preparations.

For an excellent review of fibrin sealants, see M. Brennan, Blood Reviews, 5:240-244 (1991); J. W. Gibble and P. M. Ness, Transfusion, 30:741-747 (1990); H. Matras, J. Oral Maxillofac Sura., 43:605-611 (1985) and R Lerner and N. Binur, J. of Surgical Research, 48:165-181 (1990). A major problem connected with currently used fibrin glues is the threat of transmission of infectious diseases, such as AIDS and Hepatitis B and C to a patient treated with the fibrin glue/sealant obtained from the human donors. See Opth. Surg., 23:640 (1992).

An altemate resolution to the above-mentioned risk of viral infection, advocates providing fibrinogen from a mammalian source other than a human. Fibrinogen compositions that may be provided from mammalian species other than a human are disclosed, for example, in U.S. Pat. Nos. 4,377,572 and 4,362,567. However, the therapeutic compositions defined therein are stated to contain at least about 70 mg/ml or more of fibrinogen (prior to any dilution at the site of treatment) leading potentially to the presence therein of a substantial amount of additional and antigenic protein impurities, there resulting an associated risk of severe immune response.

In view of the foregoing, practitioners of the art have sought to provide a preparation of fibrin glue that utilizes autologous fibrin, which refers to a fibrin glue in which the fibrinogen component of the fibrin glue is extracted from the patient's own blood. The use of an autologous fibrin sealant is preferred because it eliminates the risk of transmission of blood-transmitted infections, e.g., hepatitis B, non A, non B hepatitis and acquired immune deficiency syndrome (AIDS), that could otherwise be present in the fibrinogen component extracted from pooled human plasma. See L. E. Silberstein et al., Transfusion, 28:319-321 (1988); K. Laitakari and J. Luotonen, Laryngoscope, 99:974-976 (1989) and A. Dresdale et al., The Annals of Thoracic Surgery, 40:385-387 (1985). However, a substantial variation in the fibrinogen content of such preparations occurs owing to individual patient (donor) variability. Thus, a disadvantage associated with the use of such preparations is the difficulty in predicting, accurately, the clinically effective dose thereof. Accordingly, such use is of limited therapeutic value.

U.S. Pat. No. 5,185,001 discloses a method of preparing autologous plasma fibrin preoperatively to induce local hemostasis. The autologous plasma fibrin is thereafter simultaneously expelled onto a treatment site along with a physiologically acceptable thrombin solution to effect hemostasis at the site. The autologous plasma fibrin and thrombin solutions are also disclosed. Practice of that invention is limited to an autologous plasma preparation, which is contrary to the teachings of the present invention.

U.S. Pat. No. 5,407,671, EP 253 198 B1 and EP 253 198 A1 to Heimburger, et al. disclose a one-component tissue adhesive containing, in aqueous solution, fibrinogen, factor VIII, a thrombin inhibitor, prothrombin factors, calcium ions, and other components where appropriate. The Heimburger adhesive can be freeze-dried and stored until use. When the adhesive is needed, it is reconstituted to a liquid form from the freeze-dried solid by dissolving the solid in a solvent such as water. Practice of the invention described in this patent requires a combination of various components, which is contrary to the present invention.

U.S. Patent No. 5,330,974 advocates a tissue adhesive which contains fibrinogen, factor XIII, a thrombin inhibitor, prothrombin factors, calcium ions and, where appropriate, a plasmin inhibitor. The object of this invention disclosed therein lies in applying the tissue adhesive to the wound site, , wherein the components of the tissue adhesive acting in concert with accelerators which are naturally present on the wound which is to be bonded result in the thrombin which is necessary for adhesion being liberated from the prothrombin in the adhesive. Practice of this patented invention however, requires the combination of the above reference components.

U.S. Pat. No. 5,804,428, No. 5,770,194, No. 5,763,411 and No. 5,750,657 are all drawn to a fibrin sealant and methods of use thereof. The fibrin composition disclosed in the above patents contains any form of a fibrin monomer that can be converted to fibrin polymer. The thrust of the invention disclosed in the above patents is a fibrin composition which contains a fibrin 1 monomer, which is capable of spontaneously forming fibrin I polymer without the use of thrombin or factor XIII. The resulting fibrin I polymer acts as a fibrin clot.

Importantly, the source of the fibrin I monomer is irrelevant so long as the resulting fibrin I monomer is capable of converting to fibrin I polymer. Sources for the fibrin I component of the composition include blood, cell cultures that secrete fibrinogen and recombinant fibrinogen, although the blood is the preferred source. It is worth noting that practice of the invention disclosed in the above patents is limited in that it requires isolating fibrin I from either a pooled blood source or from the patient, with the latter being attended with the risk of transmission of infectious diseases. In addition, the invention is in the above patents differ from the present invention in that they each require fibrin based composition, which is contrary to the scope of the present invention.

US Patent Nos. 5,624,669 and 5,575,997 are drawn to a biocompatible monomer composition (tissue adhesive) and methods of use thereof. The biocompatible monomer composition is defined by the formula CHR.dbd.CXY ,, wherein X and Y are each strong electron withdrawing groups, and R is H, or, provided that X and Y are both cyano groups, a C.sub.1 -C.sub.4 alkyl group An example of the monomer of the inventions disclosed in the two patents is α-cyanoacrylates, which as noted infra, is attended with numerous disadvantages.

Additional fibrinogen-containing adhesive compositions and methods for the preparation thereof are provided in U.S. Pat. No. 5,804,428, No. 5,770,194, No. 5,763,411, No. 5,750,657, No. 5,510,102, No. 4,298,598, No. 4,362,567, No. 4,377,572, and No. 4,414,976.

Further disadvantages attending fibrin glues are that, to form an effective glue, the components must be kept separate from each other until the time of use, and that thrombin must be maintained at a temperature of 30.degree. C. or below.

Also, liquid-applied fibrin glues have low mechanical characteristics. In addition, formulation containing liquid fibrin glue is time consuming, and solubilizing thrombin and, more importantly, fibrinogen, is difficult.

Additionally, while fibrin glues set very rapidly, from three to five seconds, there is no increase in their adhesive strength after five minutes (J. Biomed. Mater. Res., 26:481 (1992)).

To overcome these drawbacks, fast-acting surgical adhesives have been proposed by the prior art. One group of such adhesives is the monomeric forms of alpha-cyanoacrylates.

Refer to U.S. Pat. Nos. 3,527,841 (Wicker et al.); 3,722,599 (Robertson et al.); 3,995,641 (Kronenthal et al.); and 3,940,362 (Overhults), which teach the use of α-cyanoacrylates as surgical adhesives.

Typically, when used as adhesives or sealants, cyanoacrylates are applied in monomeric form to the surfaces to be joined or sealed, where, typically, in situ anionic polymerization of the monomer occurs, giving rise to the desired adhesive bond or seal. Implants, such as rods, meshes, screws, and plates, may be formed of cyanoacrylate polymers, formed typically by radical-initiated polymerization.

However, the use of alpha-cyanoacrylate monomers and polymers *in vivo* is risky because of their potential for causing adverse tissue response. For example, methyl alpha-cyanoacrylate has been reported to cause tissue inflammation at the site of application.

For example, the use of cyanoacrylate glue following surgery as a sealant or adhesive has been determined to cause toxic effects in tissues contacted therewith resulting in tissue necrosis and foreign body immune reactions. See, for example, Epstein G. H. et al., Ann. Otol. Rhinol. Laryngol., 95, 40-45 (1986). Similarly, the use of synthetic suture materials has been reported to result in tissue ischemia and necrosis.

The adverse tissue response to α-cyanoacrylates appears to be caused by the products released during in vivo biodegradation of the polymerized alpha-cyanoacrylates. It has been proposed that formaldehyde is the biodegradation product most responsible for the adverse tissue response and, specifically, the high concentration of formaldehyde produced during rapid polymer biodegradation. Reference is made, for example, to Leonard F et al., Journal of Applied Polymer Science, Vol. 10, pp. 259-272 (1966); Leonard F, Annals, New York Academy of Sciences, Vol. 146, pp. 203-213 (1968); Tseng, Yin-Chao, et al., Journal of Applied Biomaterials, Vol. 1, pp. 111-119 (1990), and Tseng, Yin-Chao, et al., Journal of Biomedical Materials Research, Vol. 24, pp. 1355-1367 (1990). In view of the foregoing, α-cyanoacrylates have not found widespread use in hemostasis.

DEBRISAN is described as a wound cleaning bead and paste, whose use is indicated for cleaning ulcers and wounds such as venous stasis ulcers, and infected traumatic and surgical wounds. Importantly, the use of the beads is limited to cleaning a wound after it has clotted. Thus, it "teaches away" from the present invention by specifically emphasizing cleansing of the wound as opposed to promoting blood clotting and hemostasis. In addition, according to the product insert, one of the side effects of its contemplated use is "bleeding" which implies that it is not concerned with blood coagulation or hemostasis.

The aforementioned approaches and techniques for inducing blood coagulation and hemostasis all fall short of providing an effective method for treating and preventing undesired and excessive blood loss. The most significant drawback includes the use of an exogenous enzyme to facilitate the coagulation cascade. Techniques advocating the use of either autologous or nonautologous blood sources are likewise fraught with disadvantages. Importantly, none of the prior art methods teach a fibrinogen and enzyme free system for inducing rapid hemostasis.

As such, the above voids in the prior art have created an urgent need for a suitable hemostatic polymer composition which not only induces rapid blood coagulation and hemostasis at a wound or bleeding site, but also does away for the need of exogenous thrombin because of its ability to concentrate the patients own fibrinogen, which in turn, greatly facilitates the formation of a clot.

### OBJECT AND SUMMARY OF THE INVENTION

It is, therefore, a primary object of this invention to provide a novel hemostatic polymer composition for surgical and other medical purposes. In the most preferred form, the hemostatic polymer provides rapid hemostasis which allows clinicians to induce rapid blood coagulation at a wound or bleeding site, thereby allowing for the prompt and immediate adherence of the damages tissues at site of the wound.

Another aspect of the invention is that the hemostatic polymer composition significantly promotes healing of tissues in a cascade-like fashion without the use of exogenous thrombin.

Thus, in a first aspect, the invention provides hemostatic cross-linked dextran beads devoid of ionized groups for use in a method of arresting bleeding and inducing rapid blood coagulation and clot formation at a bleeding site of a mammal, as defined in the claims.

In a second aspect, the invention provides a hemostatic wound dressing comprising cross-linked dextran beads devoid of ionized groups contained in gauze, as defined in the claims.

In a third aspect, the invention relates to the use of hemostatic cross-linked dextran beads devoid of ionized groups for the manufacture of a composition for arresting bleeding and inducing rapid blood coagulation and clot formation at a bleeding site of a mammal, as defined in the claims.

Further, a method is described for treating a wound or a bleeding site in a mammal comprising applying to the wound or bleeding site a therapeutically effective amount of a hemostatic polymer composition comprising the reaction product of an uncharged substance containing organic hydroxyl groups and a bifunctional substance containing at least one of a halogen atom or an epoxy group, said bi-functional substance being reactive with the organic hydroxyl groups of the uncharged substance.

In accordance with the above method, blood coagulation and hemostasis occur upon contact of the polymer composition with blood or bleeding tissue without addition of exogenous thrombin. Blood coagulation and hemostasis occur upon contact of the hemostatic polymer composition with arterial blood flow or venous blood flow.

An alternative provides for a dry, removable storage stable, sterile wound dressing which provides a dry hemostatic zone, the dressing comprisisng a matrix containing a hemostasis-promoting amount of a therapeutic agent which accelerates blood coagulation and clot formation at an interface between a wound surface and a hemostatis promoting agent within the hemostatic zone.

An alternative method contemplates a method for promoting blood coagulation and hemostasis comprising administering to a wound or bleeding site a hemostatic polymer composition and a bioreactive agent in combination with a pharmaceutically effective carrier or diluent, the hemostatic polymer composition comprising the reaction product of an uncharged substance containing organic hydroxyl groups and a bifunctional substance containing at least one of a halogen atom and an epoxy group, in which the functional groups are reactive with organic hydroxyl groups.

Also, a pharmaceutical composition is described that is useful for rapid induction of blood coagulation and hemostasis comprising a therapeutically effective amount of a hemostatic polymer in combination with a pharmaceutically acceptable carrier or diluent, said hemostatic polymer comprising the reaction product of an uncharged substance containing organic hydmxyl groups and a bifunctional substance containing at least one of a halogen atom and an epoxy group, in which the functional groups are reactive with organic hydroxyl groups.

The pharmaceutical composition may be further combined with a bioactive agent. The bioactive agent comprises one of antibodies, antigens, antibiotics, wound sterilization substances, thrombin, blood clotting factors, conventional chemo- and radiation therapeutic drugs, VEGF, antitumor agents such as angiostatin, endostatin, biological response modifiers, and various combinations thereof. Also included are diagnostic markers.

Further, a bandage or dressing are described for inducing rapid blood coagulation and hemostasis comprising a therapeutically effective amount of a hemostatic polymer comprising the reaction product of an uncharged substance containing organic hydroxyl groups and a bifunctional substance containing at least one of a halogen atom and an epoxy group, in which the functional groups are reactive with organic hydroxyl groups.

The bandage or dressing can assume any shape or size, depending on how it is to be used. The dressing itself will preferably be flexible to be able to follow the contour of the body surface and provide full contact with the wound and surrounding area. Preferably, the wound dressing is in the form of a dry powder, gel or porous microspheres.

Also, a pharmaceutical composition is described useful for inducing rapid blood coagulation and hemostasis comprising a therapeutically effective amount of a hemostatic polymer comprising the reaction product of an uncharged substance containing organic hydroxyl groups and a bifunctional substance containing at least one of a halogen atom and an epoxy group, in which the functional groups are reactive with organic hydroxyl groups.

Further, a blood coagulating, wound healing composition is contemplated comprising a hemostatic polymer in combination with a pharmaceutically acceptable carrier or diluent, the hemostatic polymer comprising the reaction product of an uncharged substance containing organic hydroxyl groups and a bifunctional substance containing at least one of a halogen atom and an epoxy group, in which the functional groups are reactive with organic hydroxyl groups.

Alternatively, the blood coagulating, wound healing composition comprising a hemostatic polymer in combination with a pharmaceutically acceptable carrier or diluent, the hemostatic polymer comprising the reaction product of an uncharged substance containing organic hydroxyl groups and a bifunctional substance containing at least one of a halogen atom and an epoxy group, in which the functional groups are reactive with organic hydroxyl groups.

The aerosol suspension may further contain a suitable propellant selected from the group consisting of CO₂, nitrogen, air or any other suitable propellant.

Yet another embodiment is drawn to a hemostatic polymer composition further containing at least one member selected from the group consisting of collagen, fibrinogen and thrombin.

Further, a kit comprising the novel hemostatic polymer composition is described.

The above, and other objects, features and advantages of the present invention will become apparent from the description that follows.

### BRIEF DESCRIPTION OF THE DRAWINGS

Fig. 1 and Fig. la are a schematic of the hemostatic reactions. Described herein are the various reactions accruing between the cross-linked polymer that is an intrinsic feature of the hemostatic polymer composition and the platelets at the wound or bleeding site.
Fig. 2 depicts the surface reactions occurring when the hemostatic polymer composition comes in contact with the wound or bleeding site and activates the coagulation cascade.
Fig. 3 depicts the coagulation pathways occurring during the rapid coagulation of a wound or bleeding site when the hemostatic polymer composition is applied thereto.
Fig. 4 depicts an embodiment of the invention drawn to a hemostatic zone, top and side view.
Figure 5 depicts enlarged view of different matrix textures and materials for use in practicing the invention.
Fig. 6 depicts another preferred embodiment of the invention that is drawn to a bandage which includes a central portion comprisisng the hemostatic zone affixed to one face of a substrate.
Fig. 6(A) depicts another embodiment showing a hemostatic patch comprising a hemostatic zone.
Fig. 7 shows top view of a matrix separation matrix and its side view.
Fig. 8 depicts a syringe like apparatus for applying the hemostasis polymer composition of the invention.
Fig. 9 depicts an applicator gun, commonly available for applying the hemostatic accelerant (hemostatic polymer composition of the invention.
Fig. 10 depicts the use of forceps for placing a hemostatic zone onto a wound or bleeding site.
Fig. 11 depicts platelet activation by the ionic concentration of fibrinogen on the surface of the hemostatic polymer composition.

### DETAILED DESCRIPTION OF THE INVENTION

For the purpose of the subject invention, the following definitions are utilized:
"Hemostatic polymer composition" also called "hemostatic polymer" means a solution or other preparation which contains essentially two components: a substance containing uncharged organic hydroxyl groups and a substance containing at least one of a halogen atom and/or an epoxy group. The composition may also be referred to as HP 15. HP 15 means 1 gram of G-150 that swells 15 times its original volume when placed in an aqueous environment. Its molecular weight exclusion limit is 3 X 10⁵ or greater. Likewise, HP 20 is a modified form of HP 15 with lesser degree of cross-linkage. As well, its molecular weight exclusion limit is 5 X 10⁵ or greater.
"Cascade-like effect" means a sequence of reactions beginning with applying the hemostatic polymer of the invention to the wound or incision, where the hemostatic polymer rapidly triggers release of various clotting factors, and other ancillary substances, which initiate the physiological clotting process. Since the polymer is not a natural substrate for plasmin/plasminogen lytic reactions, the hemostatic reaction continues unabated until hemostasis is achieved.
"Exogenous thrombin" refers to the practice of adding exogenous thrombin to a wound site.
"Hemostatic accelerant" also refers to the hemostatic polymer composition of the invention.
"Hemostatic zone" refers to a suitable matrix containing an effective amount of the hemostatic polymer composition useful for accelerating blood coagulation and clot formation at a wound or bleeding site. It is thought that the clot formation occurs at an interface between the hemostatic zone and the wound or bleeding site surface. The clot formation is induced by the polymer composition of the invention that is contained in the matrix that forms part of the reagent zone. The dry hemostatic polymer composition of the invention can be dispersed in the matrix or applied to a surface of a matrix in an amount effective to promote and accelerate blood coagulation.
"Separation matrix" refers to the material that separates or forms a barrier between the dry hemostatic polymer composition of the invention and a surface of the wound or bleeding site.
"Bioactive" refers to any number of immunological, immuno-chemical, or chemical compositions that can be combined with the hemostatic polymer composition. Such compositions include but are not limited to: antibodies, antigens, antibiotics, wound sterilization substances, thrombin, blood clotting factors, chemo- and radiation-therapeutic drugs, gene therapy agents/substances or various combinations thereof. Also included are diagnostic markers. Gene therapy agents may include agents such as VEGF which may be needed to revascularize damaged tissue. Agents such as endostatain and angiostatin are also contemplated as gene therapy agents. Other gene therapy or wound sterilization substances may be used which are well known. including other agents kwon to one skilled in the art. Any one of the above agents may be detectably labeled with an appropriate label.
"Rapid blood coagulation" refers to the time it takes to control the bleeding at the bleeding site or for a blood clot to form or the wound site in reference to the same wound or bleeding site without the benefit of the presently claimed polymer composition. It has been surprisingly found that the disadvantages associated with conventional methods of topical application of surface coagulants such as fibrin which imitates the final phases of blood clotting mechanisms can be overcome by using the novel hemostatic polymer of the present invention.
"Co-surface" refers to the area of the reaction zone bound by the wound/bleeding site on one side and the area adjacent to, including the surface and interspacial areas essentially on the surface of the three-dimensional hemostatic polymer matrix.
"Diagnostic markers" refers to conventional markers which are well known to one skilled in the art. As examples, and without limiting the diagnostic markers to those specified, these include detectable labels including radioactive and non-radioactive labels, and photo-activated labels. Example of non-radioactive labels include the biotin/avidin system. The diagnostic labels may be useful in monitoring the course of treatment over time or the wound healing process. For example, the hemostatic polymer composition can be conjugated to a time release or bio-inert detectable marker and allowed to proceed to a wound site *in vivo* thereby allowing one to detect or image the wound over time and monitor its progress. For example the targeting of the hemostatic polymer composition can be accomplished by way of a binding agent such as an antibody that is detectably labeled. The presence of the administered hemostatic polymer composition may be detected in vitro (or ex vivo) by means known to one skilled in the art.

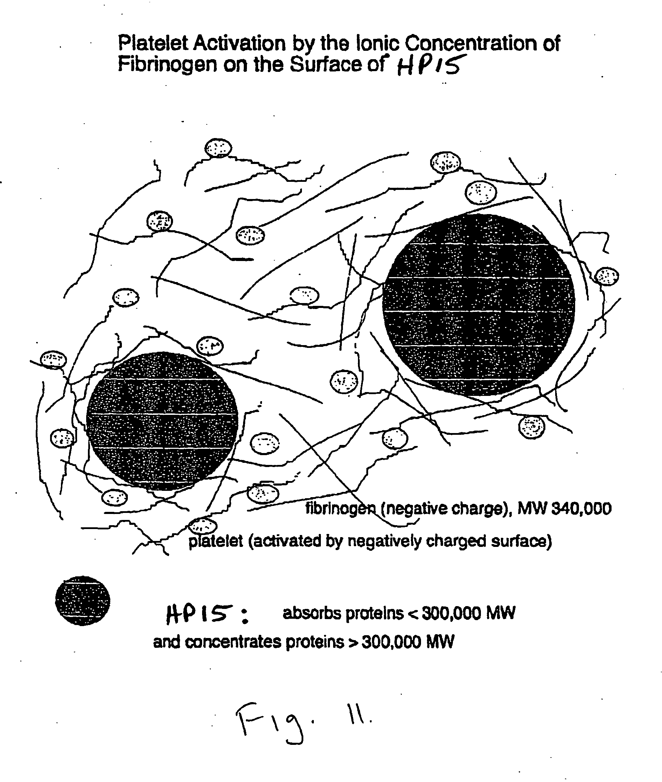

The present invention is based upon the discovery that the homeostatic polymer composition is able to induce rapid blood clotting by concentrating the patients fibrinogen *in vivo* at the site of the wound or bleeding site. The hemostatic polymer composition, acting in concert with the concentrated fibrinogen activates the patients platelets and RBC's to convert prothrombin to thrombin without the addition of exogenous thrombin. See Figure 11. It is understood that the use of the hemostatic polymer composition is not intended to be limited to the examples appearing here below. Indeed, the hemostatic polymer composition is useful for rapid blood coagulation in all mammals, including humans

Hemostasis is achieved in cascade-like fashion caused by rapid and continuous activation and aggregation of the endogenous platelets present in the patients plasma. Due to this cascade-like effect, the adhesing strength of the hemostatic polymer increases well beyond the time (3-5 minutes) during which the maximal adhesive strength is obtained physiologically or with fibrin glues, and continues until the complete hemostasis occurs.

As will be described in detail below, the novel hemostatic polymer composition of the present invention has important clinical benefits.

For example, it will find use as a tissue adhesive opposing surgically incised or traumatically lacerated tissues, sealant for preventing bleeding or for covering open wounds, system for delivering therapeutic or other bioactive agents such as antibodies, antigens, wound sterilization substances like antibiotics, analgesics, hormones, conventional chemo- and radiation-therapeutic drugs, gene therapy agents/substances, and diagnostic markers. Gene therapy agents may include agents such as VEGF which may be needed to revascularize damaged tissue. Alternatively, agents that impede angiogenesis, may also be needed at the wound or bleeding site. Thus, agents such as endostatain and angiostatin are also contemplated as being combinable with the hemostatic polymer composition of the present invention. Methods of combining any one or combinations thereof with the homeostatic polymer composition are within the skill of a skilled artisan and need not be described therein.

The homeostatic polymer composition may be used alone or in combination with other hemostatic agents such as collagen, thrombin, cationic poly-amino acids, blood clotting factors etc. to provide instant hemostasis in case of massive trauma and hemorrhage.

Thus, not only wound healing and hemostasis but also repair and regrowth of damaged tissue are contemplated.

Liquid form preparations of the polymer composition include solutions, suspensions and emulsions. As an example may be mentioned water or water-propylene glycol solutions for parenteral injection. The invention further contemplates as alternative delivery system transdermal delivery, which can take the form of creams, lotions and/or emulsions and can be included in a transdermal patch of the matrix or reservoir type as are conventional in the art for this purpose.

The pharmaceutical forms of the hemostatic composition suitable for injectable use include sterile aqueous solutions or dispersions and sterile powders for the extemporaneous preparation of sterile injectable solutions or dispersions. In all cases the form must be sterile and must be fluid to the extent that it is easy to draw into, and discharge from, a syringe.

It may be stable under the conditions of manufacture and storage and must be preserved against the contaminating action of microorganisms, such as bacteria and fungi.

The carrier can be a solvent or dispersion medium containing, for example, water, ethanol, polyol (for example, glycerol, propylene glycol, and liquid polyethylene glycol, and the like), suitable mixtures thereof, and vegetable oils. The proper fluidity can be maintained, for example, by the use of a coating, such as lecithin, by the maintenance of the required particle size in the case of dispersion and by the use of surfactants.

Solutions of the hemostatic polymer compositions can be prepared by methods known to one skilled in the art. Dispersions can also be prepared in glycerol, liquid polyethylene glycols, and mixtures thereof, and in oils. Under ordinary conditions of storage and use, these preparations contain a preservative to prevent the growth of microorganisms.

The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by use of agents delaying absorption, for example, aluminum monostearate and gelatin.

The hemostatic polymer composition is preferably administered as a sterile pharmaceutical composition containing a pharmaceutically acceptable carrier, which may be any of the numerous well known carriers, such as water, saline, phosphate buffered saline, dextrose, glycerol, ethanol, and the like, or combinations thereof. Optimization of dosages can be determined by administration of the homeostatic polymer composition and determining blood coagulation and hemostasis.

Pharmaceutical compositions for use in accordance with the present invention may be formulated in conventional manner using one or more physiologically acceptable carriers comprising excipients and auxiliaries which facilitate processing of the active composition into preparations which can be used pharmaceutically. Proper formulation is dependent upon the route of administration chosen.

For injection, the polymer composition of the invention may be formulated in aqueous solutions, preferably in physiologically compatible buffers such as Hanks's solution, Ringer's solution, or physiological saline buffer. For transmucosal administration, penetrants appropriate to the barrier to be permeated are used in the formulation. Such penetrates are generally known in the art.

Suitable routes of administration of the polymer composition may, for example, include parenteral delivery, including intramuscular, subcutaneous, intramedullary injections, as well as direct intraventricular, intravenous, intraperitoneal, intranasal, or intraocular injections; or topically. Alternately, one may administer the pharmaceutical composition comprising as its main ingredient the hemostatic polymer composition of the invention, in a local rather than systemic manner, for example, via injection of the polymer composition directly into a solid tumor. This may be accomplished in a sustained release formulation.

Preferable, the homeostatic polymer composition is administered via a two barrel syringe, with the hemostatic polymer composition being contained in one barrel while the other barrel contains thrombin, for example. The two components may be applied concomitantly or admixed prior to administration.

The homeostatic polymer composition has a long shelf life. It can be stored at or about room temperature from 2 to 5 years. In addition, the polymer composition can be carried on a person to provide instant hemostasis in case of trauma and severe hemorrhage.

The hemostatic polymer composition of the invention is prepared via a polymerization process which includes reacting an uncharged organic substance containing hydroxyl groups and a bifunctional organic substance. Details regarding the bifunctional organic substance can be found in Swedish Patent No. 865265.

Briefly, the hemostatic polymer composition is the product of a polymerization process which ultimately results in the formation of an insoluble, three-dimensional cross-linked polymer network. The resulting three-dimensional network of cross-linked polymer that defines the polymer bead or grains of the hemostatic polymer composition of the invention is formed by reacting an uncharged organic substance, containing hydroxyl group reaction sites, with either halogen or epoxy groups of a bifunctional organic substance. The three-dimensional network of cross-linked polymer may take the shape of a gel, sphere, fiber, mesh or netting when it is applied to the wound or bleeding site. A distinct feature of the bead is the presence of a three-dimensional hemostatic cascade reaction zone. The three-dimensional polymer network is further characterized as being devoid of ionized groups, insoluble in the solvent but capable of swelling in the solvent. In addition, the polymer bead of the hemostatic polymer composition is inert with regard to the substance to be isolated *in-vivo* i.e.- fibrinogen.

Briefly, the polymer composition is applied directly or with a separation matrix placed between the bleeding wound site and the hemostatic polymer composition, e.g., the separation matrix separates the hemostatic polymer composition in coming in direct contact with the wound or bleeding site surface.

Without being limited as to theory, it is likely that hemostasis occurs at the site of bleeding by the concentration of plasma proteins (i.e. fibrinogen and other clotting factors). At the start of the hemostatic cascade reaction process, depending upon the molecular dimension of the protein and the size of the pores in the three-dimensional polymer network that defines the beads of the homeostatic composition, the beads upon absorbing water, saline, plasma etc. absorb low molecular weight plasma components at the surface of the polymer beads (first layer) while concentrating higher molecular weight plasma proteins and fibrinogen just outside the first layer. The concentrated fibrinogen, in turn, forms a matrix of clotting factors, both low molecular weight and high molecular clotting factors that essentially surround the beads of the composition and also fill the interstitial space between the bead and the wound site as well as the spaces between the beads. It should be noted that beads closest to the wound site form matrixes before those farther way, and generally form the clotting matrixes as they come in contact with the blood.

Essentially, the concentrated clotting factors and the hemostatic polymer network trap platelets, which , in turn, activates the conversion of prothrombin to thrombin in the presence of Ca⁺⁺. The charged polymer, fibrinogen, and optionally collagen, exposed at the site of injury, act as binding sites for platelets and red blood cells (RBC's). The platelets undergo disruption and release thromboxane and ADP. This release induces additional platelets to adhere with the clotting factors Va, Xa, and Ca⁺⁺. This reaction, in turn, initiates the conversion of the patients' prothrombin to thrombin, which hydrolyzes four Arg-Gly peptide bonds in the purified soluble fibrinogen. The resulting long fibrin monomers spontaneously associate in forming a stable insoluble fibrin clot. As a consequence, there is no need for exogenous thrombin.

Suitable hydroxyl group-containing substances are: polyvinyl alcohol, sugar alcohol's, carbohydrates (i.e. saccharose, sorbitol), polysaccharides, (i.e. dextran, starch, alginate, cellulose), and hydroxyl group containing neutral derivatives of the above compounds.

Examples of suitable bifunctional organic substances for preparing the hemostatic polymer composition of the invention include one of epichlorohydrin, dicholorhydrin, diepoxyburan, disepoxypropyl ether, ethylene-glyco-bis-epoxypropy ether.

The co-polymerization of the organic hydroxyl group-containing substance and the bifunctional substance readily takes place in aqueous solution in the presence of an alkaline reacting catalyst. The bifunctional substance ideally contains an 1-10 atom aliphatic radical containing at least one of a halogen and epoxy reaction group, which upon reaction with hydroxyl groups yield a three dimensional cross-linked network.

### Cross-Linked Polymer and Platelet Reactions:

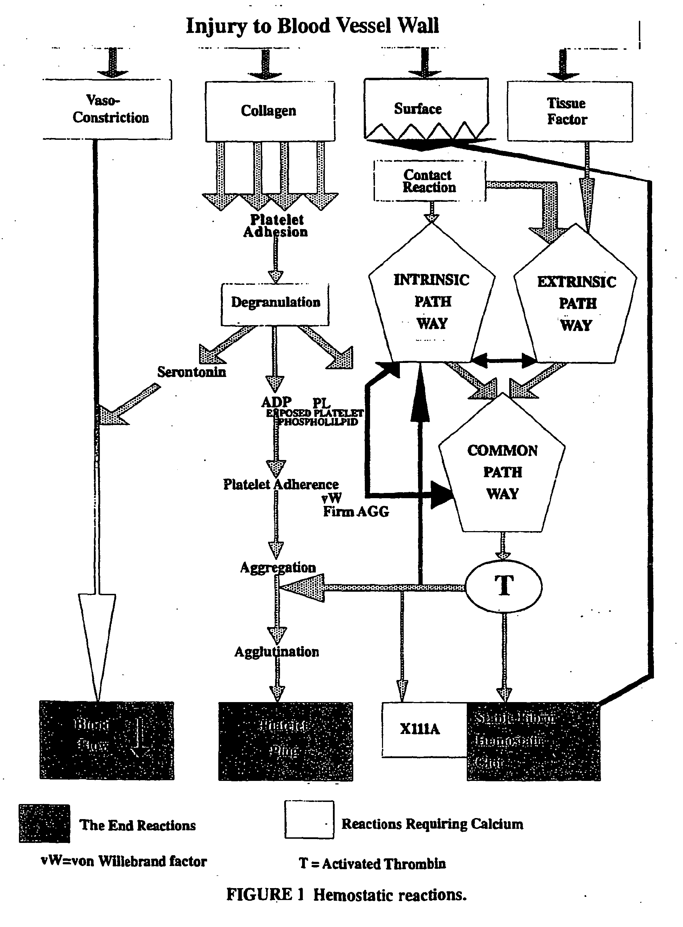

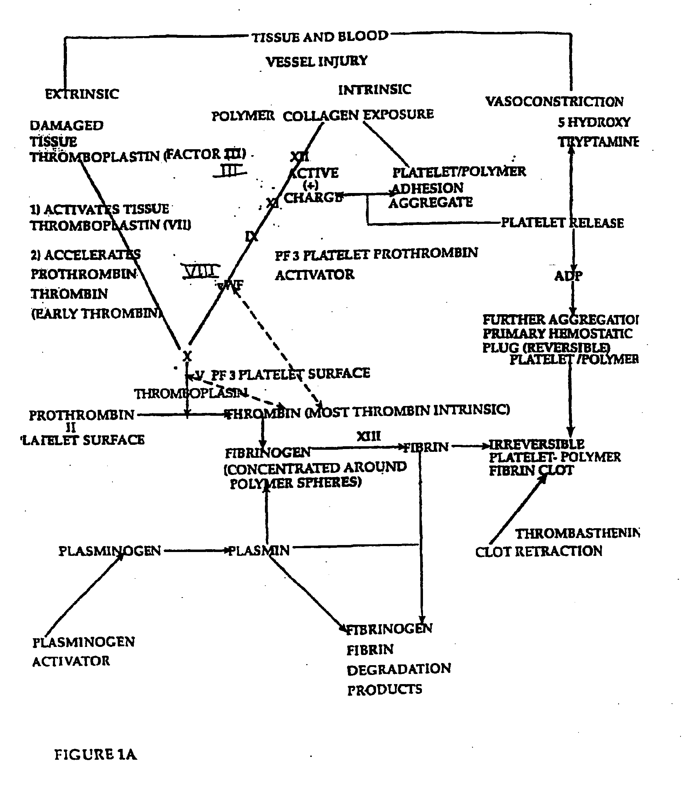

Referring to Figures 1 and 1a, shown therein are the reactions between the cross-linked polymer of the hemostatic polymer composition and blood platelets at the wound or bleeding site. The reactions between the platelets and the cross-linked polymer of the hemostatic polymer composition can be broken down into two phases, the first of which is vasoconstriction and the other is platelet plug formation.

### A. Vasoconstriction

Initially, the cross-linked polymer activates and degranulates platelets on contact, a process which ultimately leads to the release of serotonin from activated platelets as they aggregate. Serotonin, in turn, constricts the injured blood vessels and adjacent vessels in the area.

### B. Platelet plug formation

In addition to serotonin, the activation of the platelets also results in the release of ADP, and exposed platelet phospholid (Platelet Factors 1, 2 3, and 4). These platelet derived phospholipids are very important and act as a surface on which clotting factors may complex and react. ADP causes the platelets to adhere and stick to each other.

In addition, the crossed-linked polymer concentrates von Willebrand factor (vWF) (MW>800,000) in the plasma resulting in its release from the damaged endothelial cells and platelets surface. Von Willebrand factor is essential to the firm aggregation of platelets and thrombin forms the irreversible platelet agglutination (platelet plug).

### The crosse-linked polymer reaction and concentration of blood proteins:

Crossed-linked polymer beads swell with the incorporation of liquid components of the blood such as water, plasma and blood. Essentially, the beads of the invention are characterized as preventing or excluding certain molecular weight components from entering the beads, thereby effectively concentrating the excluded components outside or away from the surface of the beads,. This molecular weight exclusion limit, however, varies with the type of blood components that are absorb by the beads.

For example upon absorbing water, the exclusion limit is about 300,000. Thereafter, the limit decreases. Likewise, entry of saline lowers the exclusion limit to components having a molecular weight of about 200,000 or less. Likewise, the beads upon absorbing plasma or blood, for example, may in turn limit the entry of components having a molecular weight of less than about 100,000. Thus, the nature of the blood component that is absorbed by the beads, control, in turn, the adsorption (concentration) of clotting factors at the periphery of the beads. Thus, depending upon the type of component absorbed at the bleeding site by the beads of the hemostatic composition, varying types of blood clotting factors are adsorbed at the surface of the beads. Essentially, depending upon the size of the pores of the polymer network that defines the beads of the composition, low molecular weight plasma components enter into the beads, thereby concentrating lower molecular weight plasma proteins at essentially the surface closest to the beads, which in conjunction with higher molecular weight components of the plasma thereafter form a three-dimensional clotting matrix.

Thus, upon contacting a wound or bleeding site, the less-hydrated or dry beads of the hemostatic polymer composition effectively concentrate low molecular weight plasma components, those defined by a molecular weight of less than 300,000 (<300,000 MW), and higher molecular weight plasma components, those defined by a molecular weight of more than 300,000 (>300,000 MW) such as fibrinogen and effectively form a three-dimensional clotting matrix that essentially surrounds the beads of the composition. See Fig. 11 for example.

Factor I - fibrinogen (MW 340,000) which is highly concentrated essentially on the surface of the crossed-linked polymer beads, in turn, triggers the platelet/clotting mechanism and provides the fibrinogen for the conversion to insoluble fibrin. Thus, the concentrated fibrinogen that surrounds the crossed-linked polymer beads of the hemostatic composition acts as a high negatively charged surface for the factor XII binding and autoactivation of zymogen factor XII. High MW kininogen (Fitzgerald Factor) also binds to the high negatively charged fibrinogen surfaces. The presence of a small amount of activated XII leads to activation of its substrates, prekallikrein, factor XI, and High MW kininogen. Prekallikrein and factor XI bind to the crossed-linked polymer surface through High MW kininogen High MW kininogen also binds to prekallikrein and factor XI exists in complexes with High MW kininogen, activation of the procofactor to augment surface binding binds more prekallikrein and factor XI to the surface. On the crossed-linked polymer surface activated XII can cleave prekallikrein to kallilo-ein and activate factor XI. Kallikrein can initiate reciprocal activation, generating additional activated XII. The mechanism of reciprocal activation by crossed-linked polymer/concentrated fibrinogen is several orders of magnitude faster than autoactivation.

In summary, the crossed-linked polymer accelerates hemostasis by concentrating factors II (prothrombin MW 70,000), V (MW 330,000), VII (MW 50,000), VIII (MW 320,000), IX (MW 57,000), X (MW 59,000), X (MW 59,000) XI (MW 143,000), XII (MW 76,000), XIII (MW 320,000), High MW kininogen (Fitzgerald Factor MW 120,000 - 200,000), and Prekallikrein (Fletcher Factor MW 85,000 - 100,000).

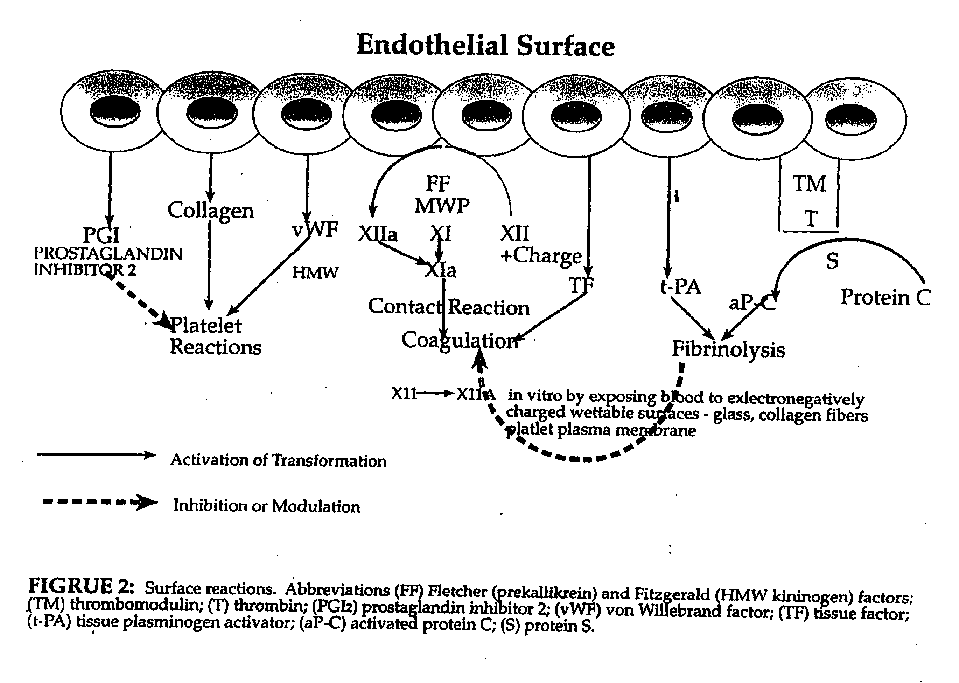

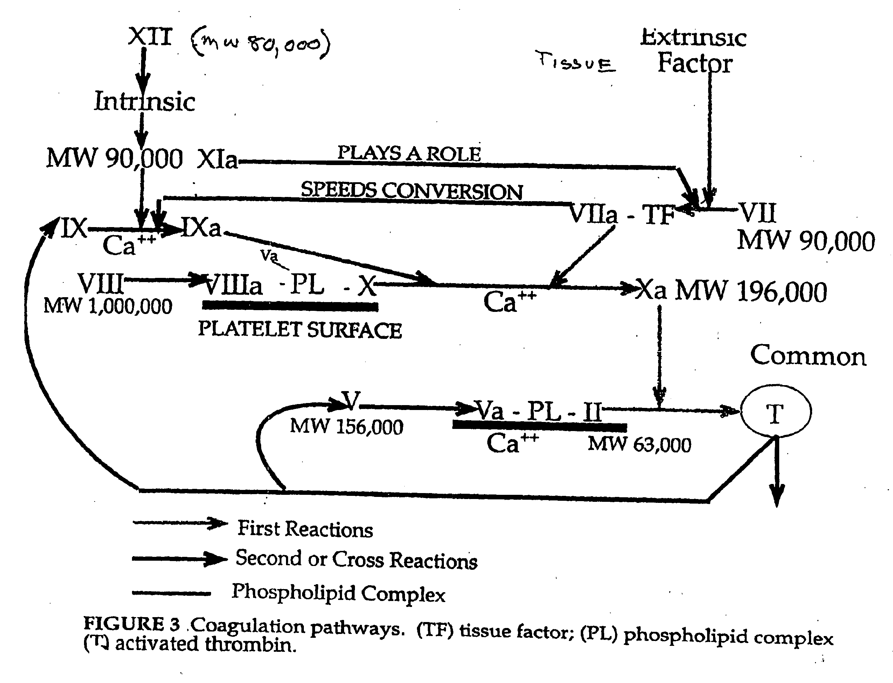

Referring to Figures 2 and 3, shown therein are the surface reactions and the coagulation pathways attending the blood clotting cascade that occurs upon administration of the hemostatic polymer composition to a wound or bleeding site.

### Coagulation Pathways and Cross-linked polymer concentrated Clotting Factors:

### A. Intrinsic pathway

Factor XII (MW 80,000) is concentrated and activated by the crossed-linked polymer. High MW kininogen (Fitzgerald factor MW 120,000 - 200,000) is also concentrated and activates additional factor XII in combination with Fletcher factor (MW 85,000 - 100,000). Factor XII activates factor XI (MW 143,000) and initiates the intrinsic pathway of coagulation. Factor XI activates factor IX (MW 57,000) and may require activated platelet phospholipid surfaces. The activation of factor IX is accelerated by the extrinsic factor VII-TF complex. Factor VIII is highly concentrated by cross-linked polymer and activated on the platelet phospholipid surface complexed with factor X. Thrombin activates the VIII, X, and platelet phospholipid complex. The intrinsic pathway combines with the extrinsic pathway to form the common pathway with the activation of factor X.

### B. Extrinsic pathway

The crossed-linked polymer also concentrates factor VII (MW 50,000) at the surface of the bead. The concentrated factor VII is activated by the released endothelial tissue factor. Factor VII is also activated by the crossed-linked polymer activated factors XII and XI. Activated factor VII also activates factor X at the end of the extrinsic pathway.

### C. Common pathway

The intrinsic and extrinsic pathways converge with the activation of factor X. These complexes become the common pathway and accelerate the conversion of concentrated factor X and factor II (prothrombin) to activated factor IIa (thrombin). The primary function of thrombin generated by the intrinsic, extrinsic and common pathways is to split two fibrino-peptides from the fibrinogen molecule, leaving the fibrin monomer that polymerizes rapidly to insoluble fibrin.

Thrombin has several additional functions including the activation of factor V on the cross-linked polymer and activated platelet phospholipid surfaces. Factor V complexes with factor II (prothrombin) on the platelet surface to generate thrombin. Thrombin activates factor XIII (MW 320,000) which cross-links the polymerized fibrin to form stable fibrin. Thrombin causes the firm agglutination of aggregated platelets into an irreversible platelet plug and fibrin clot.

Open wounds and similar body injuries which secrete or weep copious amounts of body fluid have for ever posed a formidable bandaging problem. The wound must be protected from bacterial infection, and yet provision has to be made for absorption of the body fluids that escape. A hemorrhage of a blood vessel, body tissue, organ or bone can result in blood loss leading to hypovolemic shock and death. In hemophiliacs and patients receiving anticoagulant medication, such as often prescribed post-operatively for heart surgery, the problem of rapid blood loss is even more acute.

It will be appreciated that the hemostatic polymer composition which is the essence of the novelty upon which patentability is here predicated may be applied to the wound in the various ways per se known in the art. It may be topically applied to the wound surface or packed into the wound followed by application of a protective gauze dressing or the like. Alternatively, the hemostatic polymer composition may be incorporated into a suitable matrix or substrate for application to the wound, e.g. as a coating, impregnating the matrix, or by an adhesive.

Accordingly, a dry removable hemostatic zone is provided that is removable after it has induced blood coagulation and clot formation at a wound site. The hemostatic zone consists of a suitable matrix containing effective amounts of a hemostatic agent. Preferably, the hemostatic agent comprises the hemostatic polymer composition of the invention. On the other hand, the hemostatic agent may comprises the hemostatic polymer composition of the invention in conjunction with or in addition to exogenous amounts of blood clotting components such as thrombin etc. Ideally, the hemostatic agent is in dry form. Incidentally, this novel hemostatic zone can be incorporated into any wound dressing, be it a patch, a bandage etc, where it will find use for sealing open and weeping body wounds. The hemostatic zone must contain hemostasis-promoting amounts of a hemostatic agent. Preferably, the hemostatic agent comprises the hemostatic polymer composition of the invention, which ideally is present in a dry form, although other forms of the composition may also be used.

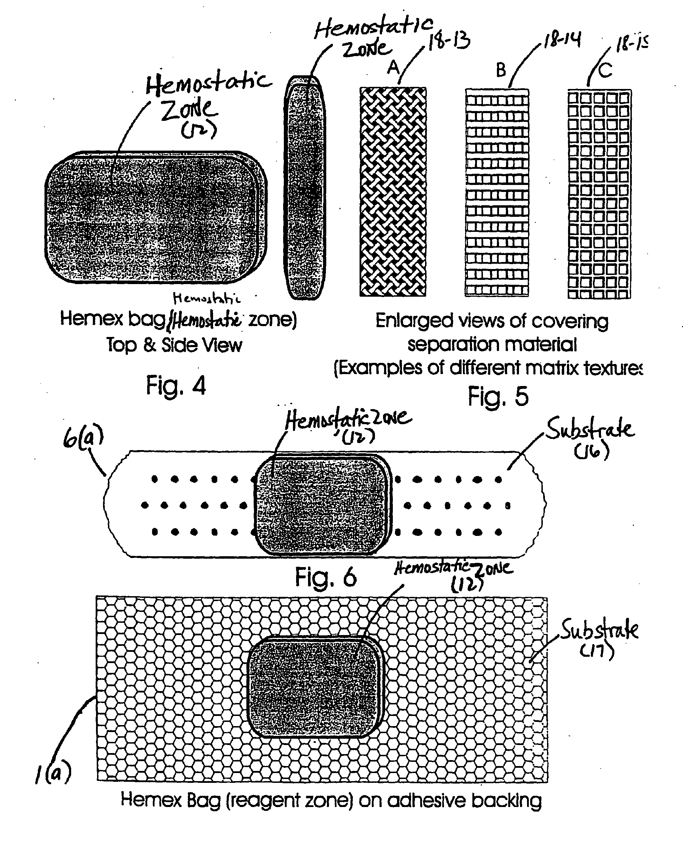

Referring to Fig. 4, shown therein is a top and side view of a dry, storage stable, sterile, removable hemostatic zone (12). A wound dressing comprising a substrate (16) carrying hemostatic zone 12 is shown in Figure 6. Herein, substrate (16) is a flexible substrate such as an adhesive Band-Aid, having a central portion consisting of hemostatic zone(12).

The hemostatic zone (12) according to the invention is made by applying to a matrix (18), a hemostasis-promoting amount of a hemostatic agent effective for accelerating blood coagulation and clot formation at an interface between a wound surface and the hemostasis promoting agent contained within the hemostatic zone. Preferably the hemostatic agent comprises the hemostatic polymer composition (hemostatic accelerant (20)) of the invention, which, in turn, comprises the reaction product of an uncharged substance containing organic hydroxyl groups and a bi-functional substance containing at least one of a halogen atom or an epoxy group, the by-functional substance being reactive with the organic hydroxyl groups of the uncharged substance.

Advantageously, the hemostatic polymer composition is applied as a layer, i.e., spraying the dry hemostatic polymer composition in powder form onto a particular surface or side of the matrix (18), which surface is then designated as the "wound-contacting surface." Alternatively, a solution of the hemostatic polymer composition can be incorporated onto or into a matrix and dried by lyophilization or by conventional means.

The dry, hemostatic zone of the invention may be of a per se known physical form for wound dressings. For instance, one useful form is as an island dressing wherein a backing or cover sheet, e.g. of a polymeric material which provides a barrier to bacteria contains a pressure-sensitive medical grade adhesive coating covering one surface thereof and a gauze or other suitable matrix containing the effective reagents of this invention is centrally disposed on the adhesive surface for application on the wound leaving free adhesive coating around the periphery of the matrix for adhering the dressing to healthy skin surrounding the wound.

On the other hand, the hemostatic polymer composition alone or as part of a hemostatic zone can be placed on a solid support, e.g., bandage, suture, prosthesis, or dressing, that will be in contact with the desired site. Such support is then placed in contact with the desired site until, for example, the fibrin clot forms. Another form preferred form is a patch.

It will be appreciated that the dry removable hemostatic zone is applied by contacting a "wound-contacting" surface of the dressing, to a wound or bleeding site surface. Then, the wound dressing is maintained in contact with the wound for a period of time sufficient for clotting to occur at the interface between the "wound-contacting surface" and the wound and for bleeding to be substantially arrested. The hemostatic zone is held in place against the biological surface preferably with light pressure. In situations where the reagent zone in/on a matrix is used to arrest bleeding at a wound or bleeding site, it may be held in place simply by applying pressure to the dressing by means of a gauze or other dry sterile material. Depending on the location of the wound, a bandage, including an elasticized bandage, can be wrapped around the reagent zone so as to provide light pressure on the wound site.

Preferably, the wound-contacting surface of the hemostatic zone is maintained in contact with the wound surface for a period of about 4-20 minutes, preferably 4-13 minutes, and most preferably from about 6 to about 10 minutes. The inventors have found that this is sufficient time for the reagent zone to accelerate the recipients blood coagulation cascade so as to form a clot at the wound or bleeding site. Thereafter, the wound dressing comprising the hemostatic zone can be removed and applied to another wound or bleeding site. The same applies to other embodiment of the invention, i.e., hemostatic patch, bandage etc, each of which carries on a suitable substrate the dry hemostatic zone as its main component.

Where the hemostatic polymer composition is applied to stabilize a wound site by temporarily arresting bleeding at the wound site, where it is separated from the wound surface by a separation matrix, the time period is preferably about 5 minutes.

A distinguishing feature of the dry removable reagent zone dressing is that, unlike conventional glues the reagent zone does not require as an ingredient any exogenous human protein, such as fibrinogen, thrombin or any other blood derived clotting factors, which in turn avoids introduction of unsafe contaminating viruses.

In addition, contrary to the teachings of conventional methods for arresting bleeding at a wound or bleeding site, the dry removable wound dressing of the invention acts a dry removable hemostatic zone which is removed after it has accelerated blood clot formation at a wound or bleeding site. This is in sharp contrast to conventional methods of wound treatment which require leaving the hemostatic agent/composition such as fibrin glue or patch etc containing the glue at a wound or bleeding site in order for the clot to form at the bleeding site. As an example, reference is had to chitosan containing bandages/wound dressings which require that the chitosan be left at the wound or bleeding site in order to induce clot formation at said site. In contrast, when the spheres of the hemostatic polymer composition swell they become larger than the pores of the matrix which are ultimately removed after a period of time.

A preferred use of a the dry hemostatic zone of the invention is to inhibit or completely stop bleeding of a parenchymal organ, such as the liver, kidney, spleen, pancreas or lungs. Additional uses for such a reagent zone, especially that which is the main component of a hemostatic patch include curbing bleeding of tissues during types of surgery such as, but not limited to, internal/abdominal, vascular (particularly for anastomosis), ufological, gynecological (particularly for an episiotomy), thyroidal, neurological, ENT, tissue transplant uses, and dental surgeries.

The matrix (18) and separation matrix are used interchangeably and contain the hemostatic polymer composition (20). Alternatively, the matrix may be a biodegradable "matrix", which as referred to herein may be employed in any of the present embodiments of the invention. It is selected from, but not limited to, the group consisting of absorbable gelatin sponge, calcium alginate, calcium/sodium alginate, collagen, and oxidized regenerated cellulose. A matrix embodying esterified collagen or chemically modified collagen is exemplified in U.S. Patent No. 4,390,519 to Sawyer. Importantly, other conventional matrices utilized in hemostatic wound dressings are contemplated for use with the novel hemostatic polymer composition of the invention. Alternatively, the matrix is a self-expandable matrix, which expands upon contacting the wound site.

One example of an advantageous matrix to which the hemostatic polymer composition and/ or other additives according to the invention are applied includes a compressible matrix. This compressed matrix self expands when in contact with an aqueous medium.

The hemostatic zone will also be useful in retarding bacterial, fungal and viral contamination and mold growth in and around a wound or bleeding site surface. This can be accomplished by admixing biological agents such as antibacterial agents etc. with the hemostatic polymer compistion prior to the admixture being dispersed or applied to a surface of the matrix.

The reagent zone may further include a biological agent for delivery to the wound or bleeding site. Thus, the reagent zone alone or in combination with a substrate, provides a mechanical barrier, a microbial barrier or a combination thereof. The reagent zone may be in the form of a wound dressing, patch, surgical barrier, bandage, or a combination thereof. The reagent zone may also be employed as a topical therapeutic formulation used with a conventional dressing, patch or Band-Aid.

The reagent zone may also include selected medicaments for local therapeutic applications. The therapeutic medicament component of the hemostatic zone may comprises a single agent such as the hemostatic polymer composition of the invention. Combination of pharmaceuticals, can be incorporated in the reagent zone or a wound dressing, as an additional layer for example.

A wound dressing comprising a suitable substrate carrying/containing the aforementioned hemostatic zone is also provided. The dry wound dressing comprising the hemostatic zone can contain as a sole agent a hemostatic agent, preferably the novel dry hemostatic polymer composition, dispersed within the matrix or applied to a surface of the matrix in an amount effective to promote and accelerate the recipients blood coagulation pathway thereby stimulating clot formation. On the other hand, the wound dressing like the hemostatic zone can contain additional therapeutic medicaments.

In one embodiment, the dry wound dressing is contained within a sealed sterile package which facilitates removal of the patch without contamination. Such a package for example, can be an aluminum foil pouch or other conventional material that is easily sterilized. Radiation, advantageously gamma radiation, is applied to sterilize the wound dressing and packaging material together. The same applies to a patch comprising the hemostatic zone.

In another embodiment, a container having dual compartments is provided. A first compartment contains a separation matrix, while the second compartment contains the hemostatic polymer composition contained in a suitable vessel, e.g., syringe. In field use, the separation matrix is applied to a wound surface and the syringe containing the dry hemostatic polymer composition is applied directly over the wound site, albeit separated by the separation matrix, for a period of time sufficient to decrease or minimize the bleeding at said site so as to provide the emergency technician/surgeon, a clearer view of the underlying trauma.

While minor cuts, bums and abrasions seldom become infected, any break in the skin can lead to localized or even systemic infection. This is of special concern in children who may not have fully developed immune systems, or in immunocompromised individuals. Accordingly, the wound dressings contemplated by the present invention will find widespread use in healing wounds in such people.

The wound dressing acting as a hemostatic zone and intended for topical applications additionally can be applied with an adhesive tape, as a Band-Aid form, where the reagent zone is adhered to an adhesive backing. Preferably the adhesive used to secure the patch is porous in areas which contact the skin. One skilled in the art is well aware of the advances in adhesive tape technology; and accordingly details of the same are omitted herein.

One or more additional layers of wound dressing material, preferably a layer which aids in absorption of blood or other exudants, can be applied to a reagent zone a/ka/a reagent bag. Such an additional layer can be made as an integral part of the zone, thereby creating a thicker zone. Alternatively, the layer may be applied as a supplement to the backside (non-wound contacting surface) of the wound dressing, e.g. a patch or flexible bandage or Band-Aid according to the invention. Particularly for topical use, the layer(s) can contain super absorbents to wick exudant solution from the wound site. It is advised that for wound dressings including those further comprising a substrate, such as a patch intended for internal-surgical applications, where an added layer(s) is integral with the patch, the layer(s) should be both biodegradable and pharmaceutically acceptable.

Therapeutic medicaments which may be used, either alone or in combination, include but are not limited to, anti-inflammatory analgesic agents, steroidal anti-inflammatory agents, antihistamines, local anesthetics, bactericides and disinfectants, vasoconstrictors, hemostatics, chemotherapeutic drugs, antibiotics, keratolytics, cauterizing agents, and antiviral drugs, hemostatic agents such as thrombin, Ca.⁺⁺ and the like, wound healing agents such as epidermal growth factor (EGF), acidic and basic fibroblast growth factors (FGFs), transforming growth factors alpha and beta (TGF alpha and beta) and the like, glycoproteins such as laminin, fibronectin and the like, various types of collagen's.

Examples of anti-inflammatory analgesic agents include acetaminophen, methyl salicylate, monoglycol salicylate, aspirin, mefenamic acid, flufenamic acid, indomethacin, diclofenac, alclofenac, diclofenac sodium, ibuprofen, ketoprofen, naproxen, pranoprofen, fenoprofen, sulindac, fenclofenac, clidanac, flurbipmfen, fentiazac, bufexamac, piroxicam, phenylbutazone, oxyphenbutazone, clofezone, pentazocine, mepirizole, tiaramide hydrochloride, etc. Examples of steroidal anti-inflammatory agents include hydrocortisone, predonisolone, dexamethasone, triamcinolone acetonide, fluocinolone acetonide, hydrocortisone acetate, predonisolone acetate, methylpredonisolone, dexamethasone acetate, betamethasone, betamethasone valerate, flumetasone, fluorometholone, beclomethasone diproprionate, etc.

Examples of antihistamines include diphenhydramine hydrochloride, diphenhydramine salicylate, diphenhydramine, chlorpheniramine hydrochloride, chlorpheniramine maleate isothipendyl hydrochloride, tripelennamine hydrochloride, promethazine hydrochloride, methdilazine hydrochloride, etc. Examples of local anesthetics include dibucaine hydrochloride, dibucaine, lidocaine hydrochloride, lidocaine, benzocaine, p-buthylaminobenzoic acid 2-(dieethylamino) ethyl ester hydrochloride, procaine hydrochloride, tetracaine, tetracaine hydrochloride, chloroprocaine hydrochloride, oxyprocaine hydrochloride, mepivacaine, cocaine hydrochloride, piperocaine hydrochloride, dyclonine, dyclonine hydrochloride, etc.

Examples of bactericides and disinfectants include thimerosal, phenol, thymol, benzalkonium chloride, benzethonium chloride, chlorhexidine, povidone iode, cetylpyridinium chloride, eugenol, trimethylammonium bromide, etc. Examples of vasoconstrictors include naphazoline nitrate, tetrahydrozoline hydrochloride, oxymetazoline hydrochloride, phenylephrine hydrochloride, tramazoline hydrochloride, etc. Examples of hemostatics include thrombin, phytonadione, protamine sulfate, aminocaproic acid, tranexamic acid, carbazochrome, carbaxochrome sodium sulfanate, rutin, hesperidin, etc.

Examples of chemotherapeutic drugs include sulfamine, sulfathiazole, sulfadiazine, homosulfamine, sulfisoxazole, sulfisomidine, sulfamediizole, nitrofiuazone, etc. Examples of antibiotics include penicillin, meticillin, oxacillin, cefalotin, cefalordin, erythromcycin, lincomycin, tetracycline, chlortetracycline, oxytetracycline, metacycline, chloramphenicol, kanamycin, streptomycin, gentamicin, bacitracin, cycloserine, etc.

Examples of keratolytics include salicylic acid, podophyllum resin, podolifox, and cantharidin. Examples of cauterizing agents include the chloroacetic acids and silver nitrate. Examples of antiviral drugs include protease inhibitors, thymadine kinase inhibitors, sugar or glycoprotein synthesis inhibitors, structural protein synthesis inhibitors, attachment and adsorption inhibitors, and nucleoside analogues such as acyclovir, penciclovir, valacyclovir, and ganciclovir.

The amount of active therapeutical medicament (s) to be used depends on the desired treatment strength and type of area to be treated.

Additionally, the wound-contacting surface of the wound dressing of the invention, e.g., hemostatic zone may be coated with a color indicator to assist the user, such as yellow vitamin B.sub.2 (riboflavin) or a suitable dye, for example, hemin. By color coding the wound-contacting surface, the user knowingly avoids touching or otherwise contaminating the wound-contacting surface of the dry wound dressing. The same applies to the other embodiments of the invention, discussed *infra*.

In addition to inducing rapid hemostasis, the inventors have found that the dry hemostatic polymer composition, is also useful for temporarily stabilizing a wound or bleeding site by temporarily retarding excessive blood flow at a profusely bleeding site. According to the above embodiment, there is provided a method for temporarily stabilizing bleeding at a wound or bleeding site, which method advocates applying, separately,
(i) a separation matrix (18) to a surface of the wound or bleeding site;
(ii) applying over the separation matrix an effective amount of a hemostatic agent to cover the wound or bleeding site; and
(iii) removing the separation matrix and the hemostatic polymer composition after the wound or bleeding site has been temporarily been stabilized as is evident from decrease in blood flow at the site.

Preferably, the hemostatic agent comprises the novel hemostatic polymer composition of the invention in dry form, although other forms of the composition can also be used. As well, other hemostatic agents can also be used, so long as these induce blood coagulation at a wound or bleeding site.

The dry hemostatic polymer composition can be applied in a simultaneous manner well known to a skilled artisan. The hemostatic polymer composition is generally contained in a suitable vessel which may include a tube having a proximal end, a distal end and a lumen extending therethrough, which contains the hemostatic polymer composition. The vessel may also include a syringe adapted to contain the novel hemostatic polymer composition or any other vessel that can be adapted to contain the hemostatic polymer composition and also be used to apply the same to a wound site, over the separation matrix. In keeping with the above embodiment, other means for temporarily stabilizing a wound or bleeding site are also contemplated. The dry hemostatic polymer composition of the invention is contained in a separation matrix (bag), where the bag acts as a suitable hemostatic zone delivery vessel. Alternatively, bandages may be used or any other device where a separation matrix is used to separate the homeostatic polymer composition from the wound or bleeding site.

The type of vessel employed depends on the choice of dispensing means and includes tubes, syringes, applicator guns, etc. The dispensing means can be manual or a pump, a fluid pressurizing component, a collapsible vessel with a tube or jet or an aerosol propellant with associated valve mechanisms. The preferred dispensing means is as a dry powder.

Alternatively, the separation matrix (18) may be affixed to an opening of the vessel containing the hemostatic polymer composition such that it is applied was a single unit to the wound and or bleeding site, with the proviso that the separation matrix separate the hemostatic polymer composition from the wound or bleeding site such that there is no direct contact between the polymer composition and the wound or bleeding site.

The separation matrix may be of the same material as the matrix that is the main component of the hemostatic zone. Indeed, in one embodiment, the separation matrix is applied to a tip of a suitable applicator, e.g., syringe and applied to a wound site. After inducing blood coagulation and hemostatis at the wound site, the separation matrix, containing a hemostasis-promoting amount of a hemostatic agent such as the dry hemostatic polymer composition may be separated from the tip of the vessel/applicator and left at the wound or bleeding site until a clot has formed at the wound site long after the vessel, containing the dry hemostatic agent has been removed. Thereafter, the separation matrix, acting a as a dry removable hemostatic zone can be removed or stripped away from the wound site after a clot is formed at the wound site.

A device for sealing an incision at a wound or blood site wherein the device contains a suitable vessel containing the hemostatic polymer composition separated at one end by a separation matrix is also described.

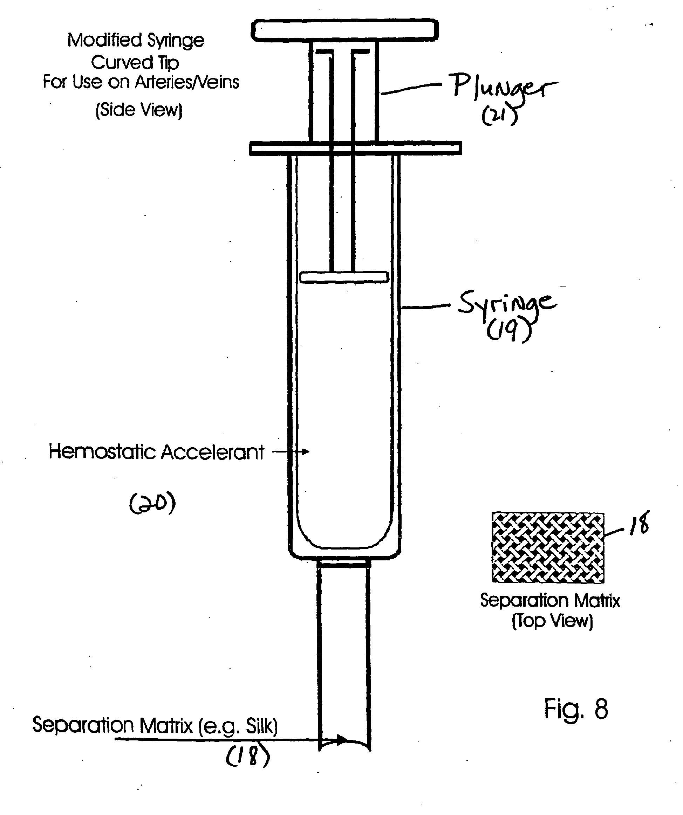

Referring to Fig. 8, shown there in is a syringe (19) containing the hemostatic polymer composition of the invention (20) and which at its opening includes a separation matrix (18) that effectively prevents the egress of the hemostatic polymer composition (hemostatic accelerant (20)) from the syringe (19). At the other end, the syringe (19) includes a plunger (21). The method advocates applying the vessel containing the homeostatic polymer composition to a wound or bleeding site for a period of time sufficient to temporality retard bleeding at said wound or bleeding site.

It is believed that as soon as the blood comes in contact with the hemostatic polymer composition of the invention, bleeding is either completely stopped to retarded to a degree to allow the medical personnel to reapply the device to another bleeding site or use other hemostatic zones on other wound or bleeding sites in a similar manner. This method will find use in various filed operations such as one where an emergency technician is presented with a patient exhibiting multiple wounds, some being more serious than others. In these situations, the technician/surgeon will be able to temporarily stabilize the wounds and find sufficient time to prioritize the preferred course of treatment after the wound sites have been stabilized. Alternatively, the hemostatic zone can be made large enough to cover large multiple bleeding site(s).

Referring to Fig. 5 (A), (B) and (C), shown therein are examples of the types of matrices 18-13, 18-14 and 18-15 than can be used in practicing the claimed invention.

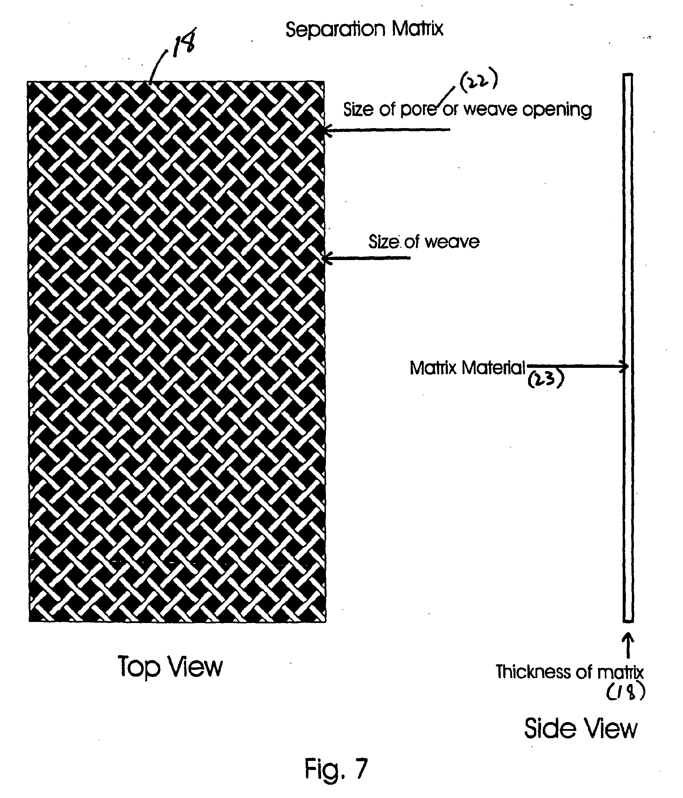

In determining what type of matrix to be used reference is had to the following. Referring to Fig. 7 shown therein is the top and side view of a suitable matrix (18) for use in practicing the invention. It will be appreciated that the micro-spheres of the hemostatic polymer composition are larger than the pore opening (22) of matrix (18). With respect to HP 15 for example, the spheres range in size from 40 to 150 micros. Thus, in the above example, the pore openings (22) of matrix (18) must be smaller than the initially dry beads (micro-spheres) of the hemostatic polymer composition. This is especially true considering that once the beads come in contact with blood at the wound or bleeding site, they swell and become larger than their initial dry size of from 40 to 150 micro, which further aids in their retention on one side of the separation matrix. The pore size of matrix (18) can be obtained by scanning electron microscope of cross-sections of fiber. The fabric thickness of the woven matrix may be the same as a single thickness of the matrix fiber, ca 25 microns.

The fibers are made up of bundles of smaller strands. The matrix fibers are generally made up of about 15 strands. The individual strands may have an irregular shape. In general, one side of the strand is preferably flatter than the other sides. Most of the individual strands are about 10 microns in width. Strands as small as 4 microns and as large as 17 microns may also be used. The fabric thickness of the matrix material (23) ranges from about 50 to about 55 microns at the intersections of the woven matrix fibers. Ideally, the separation matrix is less than 50-55 microns in thickness. Preferably, it is about 5 to about 40 microns thick, more preferably it may range in thickness from about 10 to 25 microns.

A particularly preferred composite material is a nonwoven matrix combined with a highly hydrophilic fluid absorbing material such as a polymeric absorbent fiber or particle selected from the group consisting of modified starches and high molecular weight acrylic polymers containing hydrophilic groups. Preferably, the separation matrix is composed of silk.

The inventors have also found that prior to accelerating the blood coagulation and clot formation at a wound or bleeding site, the hemostatic polymer composition of the invention also cleanses the wound. This is an important discovery considering that recently, it has been shown that the amount of moisture retained in equilibrium with wounded skin, i.e., cuts, bums and abrasions, dramatically alters the healing of the wound. It is thought that the molecules of the hemostatic polymer composition are reactive with the local environment of the wound or bleeding site surface so as to draw excess fluids, bacteria and wound exudate from the environment prior to inducing clot formation.

An improvement over fibrin glue, marketed in Europe consists of a biodegradable collagen patch onto which is impregnated bovine thrombin, aprotinin and human fibrinogen (the "TAF" patch). An example of a TAF patch is the TachoComb.RTM. patch marketed in Europe by Hafslund Nycomed Pharma, DE. The patch also contains calcium chloride to enhance coagulation. In use, this patch is removed from its package, dipped into saline solution and applied to the bleeding organ with light pressure for at least five minutes. When the bleeding has stopped, the patch is left in place by the surgeon and the cavity closed.

A major drawback to the use of fibrin glue and the TAF patch is that both contain human fibrinogen, a protein purified from human blood. Because of the high risk of HIV and hepatitis viral contamination, the Food and Drug Administration revoked the use of human fibrinogen in the United States in 1978.

Thus, an embodiment of the invention provides for an effective hemostatic patch which comprises a matrix and the hemostatic polymer composition of the invention.

According to this embodiment, there is provided a hemostatic patch suitable for rapidly arresting bleeding and inducing rapid clot formation at a wound or bleeding site, the patch comprises a dry sterile storage stable flexible matrix containing a hemostatic polymer composition on one face only thereof which provides a dry hemostatic zone. The patch is very effective in accelerating blood coagulation and clot formation at an interface between a wound or bleeding site surface and the reagent zone of the patch.

Referring to Fig. 6b, shown therein is patch (17a) comprising a flexible, adhesive substrate (17) and the hemostatic zone (12). The patch can be used externally just like a Band-Aid or dressing to a wound or bleeding site to arrest bleeding and accelerate clot formation at the wound or bleeding site. Alternatively, the patch may be used for hermetically sealing body tissue. Consider air leaking from a wound in the lungs. An efficient way of plugging or arresting the wound or bleeding site would be to apply the patch to the wound or bleeding surface , by holding the same with light pressure for example, for a period of time adequate to induce hemostasis, as discussed above. During that time, in addition to hemostasis, a hermetic seal forms. The same applies to the dry wound dressing comprising a hemostatic zone or a wound dressing comprising a hemostatic zone carried to a substrate.

Unlike conventional patches, the proposed patch of the invention does not require as an ingredient any exogenous human protein, such as fibrinogen, which thereby avoids introduction of unsafe contaminating viruses.

In general, a hemorrhage of a parenchymal organ, such as the spleen, liver, lung or pancreas, which can result from trauma or surgery, is very difficult to treat. Parenchymal organs are difficult to legate because the tissue is easily tom, pulverized or crumbled. As a result, surgeons often resort to the use of electrocautery, which can lead to further destruction of the patient's tissues. Accordingly, any one of the bandages, dressings or patches containing the hemostatic polymer composition of the invention will find use in arresting bleeding from a lesion on a parenchymal organ. Any one of the preferred wound dressings would thus be very effective in stopping bleeding in the problematic hemorrhages of parenchymal organs. In addition, the flexible matrix containing the hemostatic polymer composition,( hemostatic zone) will be easy to use and will easily mold to body contours.

Another use of a hemostatic patch includes topical treatment, such as for burn or tissue transplants. A patch intended for topical use according to the invention preferably contains additives, such as anti-infection medicaments. Bactericides, fungicides and wound healing agents can be added, as well. Neomycin and bacitracin are examples of certain additives that are incorporated into a patch intended for topical use, in addition to other therapeutic medicaments referred to above.

Another important advantage of the present invention is its flexibility, that is, the patch easily conforms to the contours of an organ or biological surface, making the manipulation of applying the patch quicker to perform. As a result, there is less overall blood loss to the patient and less time is spent in surgery.

The patch may also find use in filed situations, such as may be encountered by an emergency medical technician presented with a multiple wound patient. Therein, the patch or any other embodiment of the invention can be applied to multiple wound sites in order to effectively arrest bleeding at a wound or blood site.

The hemostatic patch like the other wound dressing comprising the hemostatic polymer composition of the invention also is useful for treating animals, preferably humans or other mammals. Thus, both companion, livestock and wild animals can be treated with any one of the embodiments of the invention.

The various wound dressings contemplated by the invention can be made to fit a particulate shape and size, which is generally dictated by its intended use.

Also, the hemostatic zone can be spherically, conically, cuboidally or cylindrically-shaped or prefabricated into small squares, such as for packing into a body cavity. Such an embodiment may find use for example, as a dental patch used for arresting bleeding in the dental cavity resulting from tooth extraction or other types of dental trauma.

The patch comprising the hemostatic zone can be designed to facilitate its application to anastomose or fuse ends of a blood vessel or other body lumen having been severed surgically or otherwise. The patch or other suitable wound dressing containing the hemostatic zone can be used in conduction with a graft used to fuse ends of a blood vessel or other lumen.

First-aid bandages are conventionally applied to superficial cuts, abrasions, punctures, sores, etc., anywhere on the body, usually in conjunction with an anti-bacterial ointment applied to an absorbent gauze pad held in place over the wound by a flexible adhesive backing material.

Over the years since the introduction of the familiar and popular Band Aid, trademark of the Johnson & Johnson Corporation, and Curad, trademark of the Kendall Corporation, improvements have been made in two basic areas: bandage materials and bandage packaging. The development of materials used in the bandages has generally improved the gauze pads' absorbency and ease of release from the wound area and the backing materials' vapor permeability and hydrophobic performance. The development of packaging has led to various designs that maintain sterility during storage and enable the user to open and apply the bandage without having to touch the adhesive backing or the absorbent gauze pad.

There currently exist two major types of bandages: the general-purpose rectangular adhesive strip in three sizes with a centrally located rectangular absorbent gauze pad, and a variety of specially shaped bandages (dots, squares, "H"-shaped and "bow tie"-shaped adhesive bandages) also having centrally located absorbent gauze pads. Conventional adhesive wound dressings usually comprise an adhesive coated sheet with a removable protector over the adhesive coating. The application of these wound dressings to a patient can be achieved by removing the protector from the adhesive sheet and adhering the sheet to a patient's skin at the wound site.

In accordance with the above, there is provided wound dressing bandage. Referring to Fig. 6A, shown therein is a bandage (16a) comprising
(i) a central portion - reagent zone (14) adapted to be directly applied to a wound or bleeding site; and
(ii) a strip (16) for adhesion to an area continuous to and in spaced-apart relation to the wound, or bleeding site, whereby the bandage is adapted to be applied substantially, without wrinkling to a contoured or flexing body part and is adapted to adhere reliably, wherein the central portion of the bandage comprises a hemostatic zone containing a suitable matrix having a hemostasis-promoting amount of a hemostatic polymer composition effective to accelerate blood coagulation and clot formation at an interface between a wound or bleeding site surface and the central portion of said bandage.

The localized treatment of body tissues, diseases, and wounds requires that the particular pharmaceutical component be maintained at the site of treatment for an effective period of time. Given the tendency of natural bodily fluids to rapidly wash away topically applied pharmaceutical components, the topical treatment of wet mucosal tissues has been problematic. In the mouth, saliva, natural replacement of the mucosal tissue, and eating, drinking, and speaking movements are typical of the problems that have limited the effectiveness and residence time of pharmaceutical carriers.

Denture adhesive pastes are well known bioadhesive products. However, these preparations are used primarily for their adhesive properties, to adhere dentures to the gums, rather than for the protection of a scab or bleeding site within the oral cavity tissue or for the topical delivery of therapeutic medicaments, although drugs such as local anesthetics may be used in the paste for the relief of sore gums. U.S. Pat. Nos. 4,894,232 and 4,518,721 describe denture adhesive pastes. Accordingly, an adhesive paste is contemplated that is adaptable for use in controlling or promoting clot formation is the oral cavity.

The use of bandages or bioadhesive laminated films, which are thinner and flexible and therefore have a decreased foreign body sensation, is also well known. Such are described in U.S. Pat. Nos. 3,996,934 and 4,286,592. These products are used to deliver drugs through the skin or mucous. The laminated films usually include an adhesive layer, a reservoir layer, and a backing layer. Accordingly, bandages or bioadhesives laminated films that can be used to seal a bleeding or wound site are described.

Bioadhesive gels, which are used for application to mucosal tissues and especially the oral cavity are also contemplated by the presently invention. Such gels can be adapted to incorporate the novel hemostatic polymer composition of the invention for use in inducing blood coagulation on mucosal tissue. For example, U.S. Pat. No. 5,192,802 describes a bioadhesive teething gel made from a blend of sodium carboxymethyl cellulose and xantham gum. Bioadhesive gels are also described in U.S. Pat. Nos. 5,314,915; 5,298,258; and 5,642,749. The gels described in those patents use an aqueous or oily medium and different types of bioadhesive and gelling agents..

In addition, film delivery systems for use on mucosal surfaces are also known. These types of systems, which are water-insoluble and usually in the form of laminated, extruded or composite films, are described in U.S. Pat. Nos. 4,517,173; 4,572,832; 4,713,243; 4,900,554; and 5,137,729. Thus, a pharmaceutical carrier device for application to mucosal surfaces to provide rapid blood coagulation and delivery of therapeutic medicaments to the site of application, surrounding tissues, and other bodily fluids, having an effective residence time is described.

Also contemplated are sutures coated with the hemostatic polymer composition of the invention. Such sutures may find use after surgery where they may be used to prevent or minimize post surgical bleeding attending some post surgical trauma.

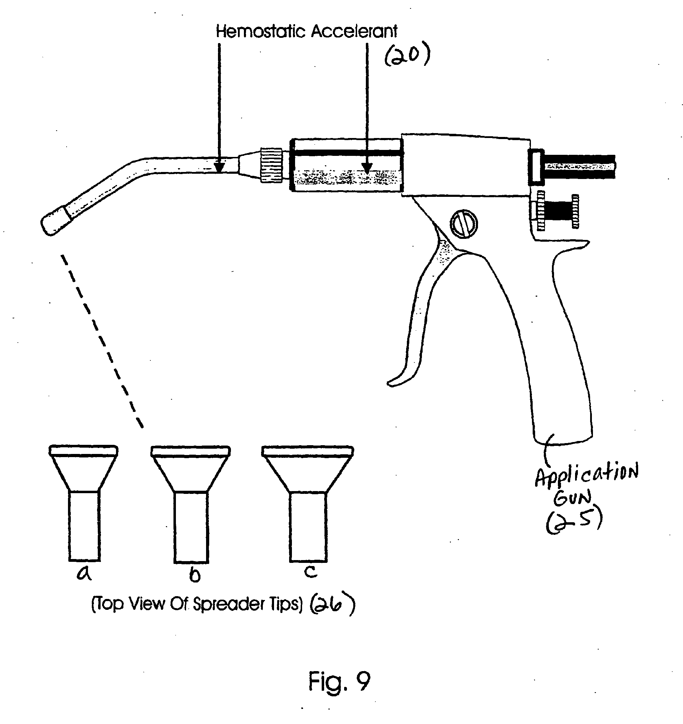

Also contemplated is a suitable vessel for delivering the dry hemostatic polymer composition of the invention to a wound or bleeding site. A preferred apparatus for the delivery of the hemostatic polymer composition acting as a hemostatic zone is shown in Fig. 9. Therein, the applicator gun (25) is shown containing the hemostatic polymer composition (20) of the invention. Also shown are the various types of spreader tips (26) (a-c) than can be used to apply the hemostatic polymer composition of the invention to a wound or bleeding site.

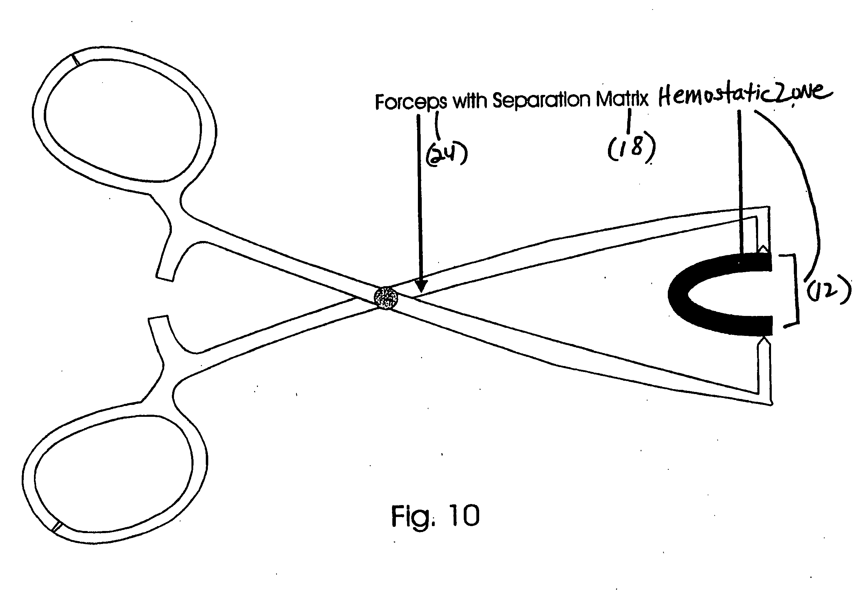

Also contemplated are means for administering the hemostatic zone (12) of the invention to for example an artery or a vein. Shown in Fig. 10 is a forceps (24) by way of which a dry hemostatic zone (12) separated by a separation matrix (18) can be effectively used to plug an artery or vein so as to accelerate blood coagulation and clot formation at an arterial or venous puncture area. Alternatively, the same apparatus can be used to temporarily stabilize multiple wounds.

The present invention is described in detail with reference to the following examples.

### Example 1

### Activation and Concentration of Platelets and Plasma Proteins by the Hemostatic Agent

Dry spheres or beads were prepared by cross-linking dextran (MW 65,000 - 70,000) with epichlorohydrin. The resulting crossed-linked dextran had exclusion limits of 100,000 MW to 300,000 MW depending on the degree of cross-linking. Ten mls of pig blood was drawn and placed in 0.1055 M buffered sodium citrate. Three tenths of a ml of the citrated blood was added to 0.05 ml of 100,000 MW, 300,000 MW and 650,000 MW cross-linked dextrans in petri dishes. The concentration of the platelets and plasma proteins were observed under a phase microscope at 200X and 400X. Within one minute, the platelets began to aggregate around the spheres. A layer of concentrated fibrinogen (fibers or strands) was observed within two minutes. Within two to five minutes, a firm fibrin clot comprised of aggregated platelets, red blood cells, and stable fibrin had formed surrounding the dextran spheres.

### Example 2

### Reduction in Clotting Time by the Hemostatic Agent

Dry spheres or beads were prepared by cross-linking dextran (MW 65,000 - 70,000) with epichlorohydrin. The resulting crossed-linked dextrans had a exclusion limit of 300,000 MW. Ten mls of sheep blood was drawn. One and a half mls of sheep blood was added to 5 tubes. Tube #1 served as the control containing citrated sheep blood only. Wet cross-linked dextran (.01 grams + 0.5 ml saline) was added to tube #2. Wet crossed-linked dextran (.01 grams + 1.0 ml saline) was added to tube #3. Dry crossed-linked dextran (.01 grams) was added to tube #4. Dry Pharmacia Dextran T70 (.01 grams, non crossed-linked) was added to tube #5. The clotting test was carried out at 39°C (normal sheep body temperature). The resulting clotting times were as follows: TUBE #1 = 14 min; TUBE #2 = 5 min; TUBE #3 = 5 min; TUBE #4 = 9.5 min; TUBE #5 = 14 min. These results demonstrate that the crossed-linked dextran (0.01 g) activated the platelets and clotting factors and reduced the clotting time by 64%.

### Example 3

### Hemostatic Effect of Cross-linked Dextran on Splenic Incision

This example illustrates the effect of the cross-linked hemostatic agent on a surgical incision of the spleen. The abdomen of a pig was surgically opened to expose the spleen. A surgical incision 6 cm long and 2 cm deep was made in the spleen. Bleeding was controlled by compression. Two grams of dry cross-linked dextran (300,000 MW exclusion limit) was placed into the incision. Hemostasis was attained by continuing the compression for 5 minutes. When the cross-linked dextran/clot was removed with forceps after 15 minutes, the spleen incision hemostasis was maintained.

### Example 4

### Hemostatic Effect of Cross-linked Dextran on Liver Trauma

This example illustrates the effect of the cross-linked hemostatic agent on experimentally induced liver trauma. A mid-line incision was made in the abdomen of a pig exposing the liver. A surgical incision 10 cm long and 3 cm deep was made in the liver. Excessive bleeding was controlled by compression. Four grams of cross-linked dextran (300,000 MW exclusion limit) was placed into the traumatized liver. Compression was continued for 5 minutes until hemostasis was attained. When the cross-linked dextran/clot was removed with forceps after 15 minutes, the liver incision hemostasis was maintained. Twelve arteries and veins had been cut and sealed by the cross-linked dextran/clot.

### Example 5

### Hemostatic Properties of the Cross-linked Dextran Hemostatic Agent on Arterial Puncture

A 100 1b pig was anesthetized and heprinized (400 units/kg). An incision was made exposing the femoral artery. A French catheter #9 was inserted into the artery via puncture through the arterial wall. A one ml syringe (cut to conform to the curved surface of the artery) containing 0.2 ml dry cross-linked dextran (300,000 MW exclusion limit) was placed over the traumatized artery and the catheter. Slow catheter removal from the puncture site with extrusion of the hemostatic agent onto the artery allowed blood to enter the syringe. As the leaking blood came in contact with the hemostatic agent the blood began to clot. The syringe rested on the artery, but care was taken not to place pressure on the femoral artery so that the flow of blood through the artery would be occluded. The syringe was slowly removed after 5 min. The arterial puncture site was sealed and hemostasis was maintained during observation for over one hour. Blood flow through the femoral artery was maintained throughout the sealing procedure.

A control arterial puncture was made in the opposite femoral artery in the same heparinized pig. The femoral artery was exposed and cleared. A French catheter #9 was inserted into the artery via puncture through the arterial wall. The fascia and skin was pulled over the catheter and puncture site and pressure was applied. With pressure being maintained the catheter was withdrawn. Bleeding could only be controlled by pressure at the puncture site resulting in cessation of blood flow through the femoral artery. Pressure was maintained for 10 min before being released, but the puncture site in the artery begin to bleed profusely. The bleeding puncture site was then sealed utilizing dry cross-linked dextran as described above.

### Example 6

A 100 1b pig was anesthetized and heprinized (400 units/kg). The object of the experiment was to test the effectiveness of the hemostatic polymer composition ( HP 15) as a hemostatic agent in an animal (pig) model with a coagulation system similar to humans. The abdomen of the pig was surgically opened to expose the spleen and liver. A surgical incision 6cm long and 2cm deep was made in the spleen. Profuse bleeding was controlled by compression. Two grams of dry HP 15 was placed into the incision. A spatula was used to apply the HP 15. The spleen was compressed together for 5 minutes.

Total hemostasis was attained in 5 minutes. After 15 - 20 minutes, the HP 15 was removed with forceps. Hemostasis was maintained, however, bleeding could be induced if the viable tissue next to the wound was cut. The dry HP 15 was very effective in attaining and maintaining hemostasis in the profusely bleeding site in the spleen.

Conclusion, the hemostatic polymer composition according to the invention and other similar crosslinking polysaccharides, etc. are very useful in arresting bleeding and accelerating clot formation at a wound or bleeding site.

A second surgical incision (10cm X 3cm deep) was made in the pigs liver. Again, profuse bleeding occurred and was controlled by 4 X 4 gauze dressing compressor. Four grams of HP 15 (4gms) was applied to the bleeding traumatized liver. Compression was applied for 5 minutes. Hemostasis was attained by the end of 5 minutes. The wound contained HP 15 was observed for 1 hour to insure that hemostasis was complete. A second wound (10cm X 3cm deep) was made in a second hole of the liver. HP 15 (4grams) was applied and hemostasis was attained in 5 minutes. After 15 minutes the clotted HP 15 was removed with forceps and the liver incision hemostasis continued to be maintained. The viable tissue on either side of the clotted wound remained well perfused and bleed profusely if cut. All of the clotted G-100 was removed and the hemostasis was maintained. When a severed artery was uncovered, it would bleed if all the clotted HP 15 was removed and the artery opened. Twelve arteries and veins had been cut and sealed using the HP 15. The severed and sealed vessels varied in size. The arteries ranged 2 mm - 5 mm. The veins ranged 2 mm -10 mm. Photographs (slides) were taken of the incision, profuse bleeding, application of HP 15. The clotting HP 15, the sealed wound, removal of HP 15 with cross sections of and the sealed vessels. The HP 15 was very effective in rapidly attaining hemostasis in liver and spleen trauma. The polymer composition of the invention appears to be biocompatible.

### Example 7

The object of this experiment was to compare the ability of conventional Avitene, Cochrum Fibrin Glue (Patent Number 5,510,102) and the dry hemostatic polymer composition of the invention; Two liver incisions 4cm X 2cm were sealed with Avitene (very poor results ). Two liver incisions were sealed with Cochrum Fibrin Glue (Patent Number 5,510,102). Although the Cochrum Fibrin Glue adhered the incision better than Avitene, the fibrin glue (plasma/polymer) however, unable to maintain hemostasis in wounds that bled profusely (arterial bleeding). The Fibrin Glue tended to stop the bleeding (due to the concentrated fibrinogen and Bovine Thrombin), however the hemostasis could not be maintained under arterial pressure.

Upon application of the hemostatic polymer composition of the invention, rapid blood coagulation was observed at the incision site. The period of time was less than that required by the Cochrum Fibrin Glue and Avitene. Also, unlike the Fibrin Glue and Avitene, hemostasis was maintained.

### Example 8

### Procedure For Femoral Access (using "Anesthetic Protocol" for pigs below) and testing of hemostatic zone ("HZ"):

A pig was anesthetized and heprinized (400 units/kg). An incision was made exposing the femoral artery. A French catheter #8 or #9 was inserted into the artery via puncture through the arterial wall. A syringe (cut to conform to the curved surface of the artery) containing dry cross-linked dextran (300,000 MW exclusion limit) was placed over the traumatized artery and the catheter. Slow catheter removal from the puncture site with extrusion of the dry hemostatic zone onto the artery allowed blood to enter the syringe. As the leaking blood came in contact with the hemostatic agent contained in and around the reagent zone, the blood began to clot. The syringe, i.e., Fig. 8 was held in place using gentle hand pressure. The syringe was slowly removed after 5 min. The arterial puncture site was continuously checked at minute intervals beginning at 5 minutes and thru 10 minutes, i.e., 5 min, 6 minutes, 7 minutes ... 10 minutes. At each minute interval after 5 minutes, the arterial puncture site was inspected and continuos clotting was observed. Thereafter, pressure was released and the area around observed for any continued hemorrhage around perimeter of the hemostatic zone, i.e., tip of syringe. Upon observing no bleeding, the syringe was gently removed from the wound site. Remaining on the wound site was the separation matrix having dispersed therein the hemostatic polymer composition of the invention, acting as a hemostatic zone. This was later teased off or gently pulled.

The protocol for the above experiment is reproduced here under. The experiment shows the successful application of a removable wound dressing which acts a dry removable hemostatic zone, which after inducing blood coagulation and clot formation at a wound or bleeding site is removed.

In the above example, the tip of the syringe which includes a separation matrix separating the dry hemostatic polymer competition from directly contacting the wound surface acts as a dry hemostatic zone, in that dispersed in the matrix are molecules of the hemostatic polymer composition, which in conjunction with the separation matrix acts as a dry hemostatic zone.

### PROTOCOL FOR ABOVE EXPERIMENT

Position the animal in dorsal recumbency, Retract the rear right leg caudally.
Shave the surgical access site.
Approach the femoral artery with a longitudinal incision over the fascial division of the sartorius and gracilis muscles. Separate the musculature and isolate the segment of femoral artery located below the edge of the gracilis muscle.
The femoral artery may be wrapped loosely with suture in order to isolate the vessel and facilitate its manipulation.
Stop flow on artery by pulling up on sutures.
Make a small incision with Iris scissors 1-2mm and deep enough to penetrate artery wall (arteriotomy).
Introduce an 8 or 9 French catheter via the arteriotomy into the artery lumen to assure opening, release sutures then extract catheter creating a bleeding wound site.
Place the syringe (see: Fig 8) carrying the HZ over the wound site as the catheter is withdrawn. Hold syringe in place using gentle hand pressure.
Check site at minute intervals beginning at 5 minutes (5, 6, 7, 8, 9, ... 10 minutes). (**At each of these times, clotting was observed.)**
Release the hand pressure.
Observe for any continued hemorrhage around the perimeter of the HZ (tip area of syringe).
If none, gently pull syringe off the wound site leaving the HZ in place on the wound site.
The HZ may be removed at a latter time by "teasing" or gently peeling the HZ off the wound site from one end to the other.

### ANESTHETIC PROTOCOL - PIG

### BODY WEIGHT:

25 to 90kg.

### PREMEDICATE:

Atmpine 0.5mg/10kg (not to exceed 1.5mg) I M.
Acepromazine 1 mg/10kg (not to exceed 5.0mg) I.M.

### INDUCED ANESTHESIA:

Ketamine HCI I 5mg/kg I.M. (may be repeated in half doses as necessary).
Xylazine 20-80mg I.M, in pigs over 40kg.
Isoflurane 3.5% mask induction.

### MAINTENANCE ANESTHESIA:

Isoflurane via endotracheal tube (2.0%-3.0% usually).

### ANTICOAGULANT (When needed):

Heparin at 300 units/kg BW initially. Check ACT's every 30 minutes and give repeat
Heparin as needed, usually 150 units/kg at 30 minute intervals. Keep ACT's above 400.

### RECOVERY:

Keep warm and comfortable, and on sternum. Butorphanol 0.1 to 0.3mg/kg I.M. every 4 hours if needed. Antibiotics as instructed by the veterinarian.

Disposition:
In house for short term care Contract outside facility for long term care.

### FLUIDS:

Normal Saline Solution via ear vein (or medial metacarpal or metatarsal vein).
Moderate drip, usually 500~1000cc per procedure or as needed, especially in heart catheter procedures.

### EUTIIANASIA:

While under anesthesia' give 10-20cc rapid I.V. injection of concentrated
(2mEq/ml) KCI.

### Example 9

**Procedure for Abdominal Access (using "Anesthetic Protocol" for pigs below) and testing of hemostatic zone ("bag"):**
Position the animal in dorsal recumbency.
Shave the abdominal region for surgical access.
Expose the abdominal cavity with a ventral midline incision from the xiphoid to the pubis.
Position a Balfour retractor to facilitate access to the liver, spleen and descending aorta. Moist gauze and surgical towels should be used to protect the organs and tissues of the abdomen.
Make an incision roughly 7-9 cm long and 1.5-2 cm deep using a No. 20 surgical scalpel blade
Assure that the site is bleeding freely.
Blot the site with gauze then place the bag immediately on the site.
Hold the bag on the site with gentle hand pressure.
Check site at minute intervals beginning at 5 minutes (5, 6, 7, 8, 9, ... 12 minutes). **(At each of these times, clotting was observed.)**
Release the hand pressure.
Observe for any continued hemorrhage around the perimeter of the bag.
If none, gently remove bag by "teasing" or gently peeling the bag off the wound from one end to the other.

### ANESTHETIC PROTOCOL - PIG

### BODY WEIGHT:

25 to 90kg.

### PREMEDICATE:

Atropine 0.5mg/10kg (not to exceed 1.5mg) I M.
Acepromazine 1 mg/10kg (not to exceed 5.0mg) I.M.

### INDUCED ANESTHESIA:

Ketamine HCI I 5mg/kg I.M. (may be repeated in half doses as necessary).
Xylazine 20-80mg I.M, in pigs over 40kg.
Isoflurane 3.5% mask induction.

### MAINTENANCE ANESTHESIA:

Isoflurane via endotracheal tube (2.0%-3.0% usually).

### ANTICOAGULANT (When needed):

Heparin at 300 units/kg BW initially. Check ACT's every 30 minutes and give repeat
Heparin as needed, usually 150 units/kg at 30 minute intervals. Keep ACT's above 400.

### RECOVERY:

Keep warm and comfortable, and on stemum. Butorphanol 0.1 to 0.3mg/kg I.M.
every 4 hours if needed. Antibiotics as instructed by the veterinarian.

Disposition:
In house for short term care Contract outside facility for long term care.

### FLUIDS:

Normal Saline Solution via ear vein (or medial metacarpal or metatarsal vein).
Moderate drip, usually 500~1000cc per procedure or as needed, especially in heart catheter procedures.

### EUTHANASIA:

While under anesthesia' give 10-20cc rapid I.V. injection of concentrated
(2mEq/ml) KCI.

### Example 10

This experiment demonstrates the use of a hemostatic zone in inducing blood coagulation at a wound or bleeding site wherein the reagent zone comprises a matrix containing the novel dry hemostatic polymer composition of the invention together with added thrombin. Dry bead size of the spheres of the composition were from 10 to 120 microns. Thrombin: Dry lyophilized bovine thrombin (dry flake appearance). The dry thrombin was used 500 units per 0.5 g of the hemostatic polymer composition of the invention. Thrombin USP Parke-Davis 5000 units/vial.

The procedure was the same as in example 9 except that the hemostatic agent included exogenously added thrombin. A similar experiment using the hemostatic polymer composition of the invention in conjunction with bovine collagen provided similar results when used to seal a femoral artery of a pig. Therein, 0.01 ml Avitene was used with 0.2 ml of the polymer composition to prevent the polymer composition from falling out of the syringe. The bovine collagen was used as a separation matrix.

Dry thrombin mixed with Hemex - 1 part thrombin poured onto 10 parts Hemex in a tube. Tube then agitated (shaked) for 30-60 seconds. Mixture was then placed into the hemostatic zone (HZ) bag.

**Procedure for abdominal access (using "anesthetic protocol" for pigs) and testing of hemostatic zone ("bag"):**
Position the animal in dorsal recumbency.
Shave the abdominal region for surgical access.
Expose the abdominal cavity with a ventral midline incision from the xiphoid to the pubis.
Position a Balfour retractor to facilitate access to the liver, spleen and descending aorta. Moist gauze and surgical towels should be used to protect the organs and tissues of the abdomen.
Make an incision roughly 7-9 cm long and 1.5-2 cm deep using a No. 20 surgical scalpel blade
Assure that the site is bleeding freely.
Blot the site with gauze then place the bag immediately on the site.
Hold the bag on the site with gentle hand pressure.
Check site at minute intervals beginning at 5 minutes (5, 6, 7, 8, 9, ... 12 minutes). (At **each of these times, clotting was observed.)**
Release the hand pressure.
Observe for any continued hemorrhage around the perimeter of the reagent zone (bag).
If none, gently remove bag by "teasing" or gently peeling the bag off the wound from one end to the other. The HZ is separated from the wound site after this procedure.

### Example 11

The following example illustrates the use of the novel hemostatic polymer composition of the invention i.e., application of the hemostatic polymer composition (i.e., HP 15) for controlling bleeding in a human. A subject was observed with a cut on the tip of a middle finger. The cut measured from about 8 to about 9 mm in length and bled profusely. The wound was allowed to bleed freely for several minutes, and when it did not stop bleeding , a small amount of the hemostatic polymer composition (dry HP 15) was applied to the bleeding surface of the wound. A small bandage was applied over the wound and the polymer compistion. Bleeding appeared to stop immediately. After about 20 to 45 minutes, the bandage was removed from the wound site and the wound observed. It was noticed that the wound was covered by a blood-polymer clot. The clot appeared to adhere well to the surrounding skin. The polymer composition was saturated with blood that had coagulated forming a flexible clot which appeared to protect the wound. The resulting clot material was somewhat resistant to removal and was washed off under a running stream of warm water. Importantly, upon removal of the clot material, the wound did not start bleeding again. A clean bandage was applied to the wound and it healed without event. Characteristics of the clot seemed very similar to that observed with pig blood.

### Example 12

This experiment demonstrates the bio-compatibility HP 15 and HP 20 (cross-linked polysaccharide) in skin incisions in a sheep model.

Four skin incisions (#1 - #4) were made in and around the left flank of an anesthetized sheep.

Incision 1 and 2 were treated with hemostatic promoting amounts of HP 15 to stop bleeding. Incision 3 was treated with similar amount of HP 20, while incision #4 was left untreated (control). Two sutures (5-0 Dermalon) were used to prevent skin from opening since the sheep would be very active when conscious and awake.

Note: 1 incision had a 1 cm hematoma which was caused by the cutting needle of the 5-0 Dermalon.

HP 15 and Hp 20 were observed to be very effective hemostatic agents in sealing the skin incision. Subsequent histological slides of sheep skin treated with the above agents were studies and confirmed the following. Incision #1 (HP 15) and #2 (HP 20) were completely healed in the histological section. The HP 15 remained in the tissue however, and the spheres appeared to biodegrade and were surrounded by minimal mononuclear cells. There was no sign of a host reaction to the HP 15.

Incision #3 (HP 20 treated) exhibited the same results as the wound treated with HP 15, i.e., the histological examination revealed a similar histology. However, it appeared that HP 20 was more biodegradable than HP 15. Also, HP 20 like HP 15 was very biocompatible and the slides did not show any host reaction towards it.

## Claims

1. Hemostatic cross-linked dextran beads devoid of ionized groups for use in arresting bleeding and inducing rapid blood coagulation and clot formation at a bleeding site of a mammal.

2. The beads as claimed in claim 1, wherein said mammal is a human.

3. The beads as claimed in claims 1 or 2, wherein said bleeding site comprises a break in the skin or wounded skin.

4. The beads as claimed in claims 1 or 2, wherein said bleeding site comprises a wound to a parenchymal organ, particularly wherein said organ comprises the liver, kidney, spleen, pancreas, or lungs.

5. The beads as claimed in claim 1, wherein said beads are contained in a matrix selected from the group consisting of absorbable gelatin sponge, calcium alginate, calcium/sodium alginate, and oxidized regenerated cellulose.

6. The beads as claimed in claim 1, wherein said beads are contained in a gauze matrix.

7. The beads as claimed in any of the preceding claims, wherein said beads are contained in a matrix which further contains a pharmaceutical agent, particularly wherein said pharmaceutical agent comprises at least one of anti-inflammatory analgesic agents, steroidal anti-inflammatory agents, antihistamines, local anesthetics, bactericides or disinfectants, vasoconstrictors, chemotherapeutic drugs, antibiotics, keratolytics, cauterizing agents, antiviral drugs, and mixtures thereof.

8. A hemostatic wound dressing comprising cross-linked dextran beads devoid of ionized groups as claimed in claim 1 contained in gauze.

9. The hemostatic wound dressing of claim 8,
(a) wherein said gauze is disposed on the wound dressing via an adhesive;
(b) wherein said beads are contained in gauze as a consequence of having been sprayed on said gauze;
(c) wherein said beads have a molecular weight exclusion limit of 3 X 10⁵ or greater;
(d) wherein said beads have a molecular weight exclusion limit of 5 X 10⁵ or greater;
(e) wherein said wound dressing further contains a pharmaceutical agent, particularly wherein said pharmaceutical agent is selected from the group consisting of anti-inflammatory analgesic agents, steroidal anti-inflammatory agents, antihistamines, local anaesthetics, bactericides, disinfectants, vasoconstrictors, chemotherapeutic drugs, antibiotics, keratolytics, cauterizing agents, antiviral drugs, and mixtures thereof; or
(f) wherein said wound dressing further contains at least one of collagen, fibrinogen, and thrombin.

10. Use of hemostatic cross-linked dextran beads devoid of ionized groups for the manufacture of a composition for arresting bleeding and inducing rapid blood coagulation and clot formation at a bleeding site of a mammal.

## Patentansprüche

1. Hämostatische, querverknüpfte Dextrankügelchen, frei von ionisierten Gruppen, zur Verwendung in dem Stoppen von einer Blutung und in dem Induzieren von rascher Blutgerinnung und Gerinnselbildung an einer Blutungsstelle eines Säugers.

2. Kügelchen, wie in Anspruch 1 beansprucht, wobei der Säuger ein Mensch ist.

3. Kügelchen, wie in Ansprüchen 1 oder 2 beansprucht, wobei die Blutungsstelle einen Riss in der Haut oder verwundete Haut umfasst.

4. Kügelchen, wie in Ansprüchen 1 oder 2 beansprucht, wobei die Blutungsstelle eine Wunde eines parenchymalen Organs umfasst, insbesondere wobei das Organ die Leber, Niere, Milz, Pankreas oder Lungen umfasst.

5. Kügelchen, wie in Anspruch 1 beansprucht, wobei die Kügelchen in einer Matrix enthalten sind, ausgewählt aus der Gruppe bestehend aus absorbierbarem Gelatineschwamm, Calciumalginat, Calcium-/Natriumalginat und oxidierter regenerierter Cellulose.

6. Kügelchen, wie in Anspruch 1 beansprucht, wobei die Kügelchen in einer Gazematrix enthalten sind.

7. Kügelchen, wie in einem beliebigen der vorhergehenden Ansprüche beansprucht, wobei die Kügelchen in einer Matrix enthalten sind, welche des Weiteren ein pharmazeutisches Mittel enthält, insbesondere wobei das pharmazeutische Mittel mindestens eines von antiinflammatorischen Analgetika, steroiden antiinflammatorischen Mitteln, Antihistaminika, Lokalanästhetika, Bakteriziden oder Desinfektionsmitteln, Vasokonstriktoren, chemotherapeutischen Arzneimitteln, Antibiotika, Keratolytika, kauterisierenden Mitteln, antiviralen Arzneimitteln und Mischungen davon umfasst.

8. Hämostatischer Wundverband, umfassend wie in Anspruch 1 beanspruchte querverknüpfte Dextrankügelchen, frei von ionisierten Gruppen, enthalten in Gaze.

9. Hämostatischer Wundverband nach Anspruch 8,
(a) wobei die Gaze auf dem Wundverband über ein Haftmittel angeordnet ist;
(b) wobei die Kügelchen als eine Konsequenz dessen, dass sie auf die Gaze aufgesprüht worden sind, in Gaze enthalten sind;
(c) wobei die Kügelchen eine Molekulargewicht-Ausschlussgrenze von 3 X 10⁵ oder größer haben;
(d) wobei die Kügelchen eine Molekulargewicht-Ausschlussgrenze von 5 X 10⁵ oder größer haben;
(e) wobei der Wundverband weiterhin ein pharmazeutisches Mittel enthält, insbesondere wobei das pharmazeutische Mittel ausgewählt ist aus der Gruppe bestehend aus antiinflammatorischen Analgetika, steroiden antiinflammatorischen Mitteln, Antihistaminika, Lokalanästhetika, Bakteriziden, Desinfektionsmitteln, Vasokonstriktoren, chemotherapeutischen Arzneimitteln, Antibiotika, Keratolytika, kauterisierenden Mitteln, antiviralen Arzneimitteln und Mischungen davon; oder
(f) wobei der Wundverband des Weiteren mindestens eines von Kollagen, Fibrinogen und Thrombin enthält.

10. Verwendung von hämostatischen querverknüpften Dextrankügelchen, frei von ionisierten Gruppen, für die Herstellung einer Zusammensetzung zum Stoppen von einer Blutung und zum Induzieren von rascher Blutgerinnung und Gerinnselbildung an einer Blutungsstelle eines Säugers.

## Revendications

1. Perles de dextrane réticulées hémostatiques dépourvues de groupes ionisés pour leur utilisation pour arrêter un saignement et induire une coagulation sanguine rapide et une formation de caillot au site de saignement d'un mammifère.

2. Perles selon la revendication 1, où ledit mammifère est un humain.

3. Perles selon la revendication 1 ou 2, où ledit site de saignement comprend une coupure dans la peau ou de la peau blessée.

4. Perles selon la revendication 1 ou 2, où ledit site de saignement comprend une blessure à un organe parenchymateux, en particulier où ledit organe comprend le foie, le rein, la rate, le pancréas, ou les poumons.

5. Perles selon la revendication 1, où lesdites perles sont contenues dans une matrice choisie dans le groupe constitué d'éponge de gélatine absorbable, d'alginate de calcium, d'alginate de calcium/sodium, et de cellulose régénérée oxydée.

6. Perles selon la revendication 1, où lesdites perles sont contenues dans une matrice de gaze.

7. Perles selon l'une quelconque des revendications précédentes, où lesdites perles sont contenues dans une matrice qui contient en outre un agent pharmaceutique, en particulier où ledit agent pharmaceutique comprend au moins l'un parmi des agents analgésiques anti-inflammatoires, des agents anti-inflammatoires stéroïdiens, des antihistaminiques, des anesthésiques locaux, des bactéricides ou des désinfectants, des vasoconstricteurs, des médicaments chimiothérapeutiques, des antibiotiques, des kératolytiques, des agents de cautérisation, des médicaments antiviraux, et leurs mélanges.

8. Pansement pour blessure hémostatique comprenant des perles de dextrane réticulées dépourvues de groupes ionisés selon la revendication 1 contenues dans de la gaze.

9. Pansement pour blessure hémostatique selon la revendication 8,
(a) dans lequel ladite gaze est disposée sur le pansement pour blessure via un adhésif ;
(b) dans lequel lesdites perles sont contenues dans de la gaze après avoir été pulvérisées sur ladite gaze ;
(c) dans lequel lesdites perles ont une limite d'exclusion de masse moléculaire de 3 X 10⁵ ou supérieure ;
(d) dans lequel lesdites perles ont une limite d'exclusion de masse moléculaire de 5 X 10⁵ ou supérieure ;
(e) dans lequel ledit pansement pour blessure contient en outre un agent pharmaceutique, en particulier dans lequel ledit agent pharmaceutique est choisi dans le groupe constitué d'agents analgésiques anti-inflammatoires, d'agents anti-inflammatoires stéroïdiens, d'antihistaminiques, d'anesthésiques locaux, de bactéricides, de désinfectants, de vasoconstricteurs, de médicaments chimiothérapeutiques, d'antibiotiques, de kératolytiques, d'agents de cautérisation, de médicaments antiviraux, et leurs mélanges ; ou
(f) dans lequel ledit pansement pour blessure contient de plus au moins l'un parmi du collagène, du fibrinogène, et de la thrombine.

10. Utilisation de perles de dextrane réticulées hémostatiques dépourvues de groupes ionisés pour la fabrication d'une composition destinée à arrêter un saignement et à induire une coagulation sanguine rapide et une formation de caillot au site de saignement d'un mammifère.
